# EUROPÄISCHE PATENTSCHRIFT

(11) **EP 2 021 800 B1**
(45) Veröffentlichungstag und Bekanntmachung des Hinweises auf die Patenterteilung: **20.07.2011**
(21) Anmeldenummer: 07725061.1
(22) Anmeldetag: 10.05.2007
(51) Int. Cl.: G01N 33/542, C12Q 1/00, C12Q 1/34, C12Q 1/527

(54) **BESTIMMUNG VON KONZENTRATIONSÄNDERUNGEN**
DETERMINATION OF CHANGES IN CONCENTRATION
DETERMINATION DE CHANGEMENTS DE CONCENTRATION

(30) Priorität: 16.05.2006 DE 102006023083
(43) Veröffentlichungstag der Anmeldung: 11.02.2009
(73) Patentinhaber: Jacobs University Bremen GmbH, 28759 Bremen (DE)
(72) Erfinder: NAU, Werner, 28717 Bremen (DE); BAKIRCI, Huseyin, F-67200 Strasbourg (FR); HENNIG, Andreas, 38268 Lengede (DE)
(74) Vertreter: Castell, Klaus
(86) Internationale Anmeldenummer: PCT/EP2007/004139
(87) Internationale Veröffentlichungsnummer: WO 2007/131696

(56) Entgegenhaltungen:
- EP-A1- 0 010 615
- EP-A1- 0 010 615
- BAKIRCI HUSEYIN ET AL: "Fluorescence regeneration as a signaling principle for choline and carnitine binding: A refined supramolecular sensor system based on a fluorescent azoalkane" ADV. FUNCT. MATER.; ADVANCED FUNCTIONAL MATERIALS JAN 19 2006, Bd. 16, Nr. 2, 19. Januar 2006 (2006-01-19), Seiten 237-242, XP002446124
- GALE P A ET AL: "A colourimetric calix[4lpyrrole-4-nitrophenolate based anion sensor" CHEMICAL COMMUNICATIONS 21 SEP 1999 UNITED KINGDOM, Bd. 5, Nr. 18, 21. September 1999 (1999-09-21), Seiten 1851-1852, XP002446125 ISSN: 1359-7345
- INOUYE MASAHIKO ET AL: "Nondestructive detection of acetylcholine in protic media: Artificial-signaling acetylcholine receptors" JOURNAL OF THE AMERICAN CHEMICAL SOCIETY, Bd. 116, Nr. 12, 1994, Seiten 5517-5518, XP002446126 ISSN: 0002-7863
- ATILGAN, SERDAR ; AKKAYA, ENGIN U: "A calixpyridinium-pyranine complex as a selective anion sensing assembly via the indicator displacement strategy" TETRAHEDRON LETTERS, Bd. 45, Nr. 50, 2004, Seiten 9269-9271, XP002446127
- HENNIG ANDREAS ET AL: "Effects of cucurbit[7]uril on enzymatic activity." CHEMICAL COMMUNICATIONS (CAMBRIDGE, ENGLAND) 28 APR 2007, Nr. 16, 28. April 2007 (2007-04-28), Seiten 1614-1616, XP009087927 ISSN: 1359-7345
- WISKUR S L ET AL: "Teaching old indicators new tricks." ACCOUNTS OF CHEMICAL RESEARCH DEC 2001, Bd. 34, Nr. 12, Dezember 2001 (2001-12), Seiten 963-972, XP002446129 ISSN: 0001-4842
- ABRAHAM, W: "Inclusion of Organic Cations by Calix [n]arenes" JOURNAL OF INCLUSION PHENOMENA AND MACROCYCLIC CHEMISTRY, Bd. 43, Nr. 3-4, 2002, Seiten 159-174, XP002446130
- HENNIG ANDREAS ET AL: "Label-free continuous enzyme assays with macrocycle-fluorescent dye complexes." NATURE METHODS AUG 2007, Bd. 4, Nr. 8, August 2007 (2007-08), Seiten 629-632, XP009088178 ISSN: 1548-7091
- BAILEY DAVID M ET AL: "Supramolecular tandem enzyme assays for multiparameter sensor arrays and enantiomeric excess determination of amino acids.", CHEMISTRY (WEINHEIM AN DER BERGSTRASSE, GERMANY) 2008, vol. 14, no. 20, 2008, pages 6069-6077, ISSN: 0947-6539
- NAU WERNER M ET AL: "Substrate-Selective Supramolecular Tandem Assays: Monitoring Enzyme Inhibition of Arginase and Diamine Oxidase by Fluorescent Dye Displacement from Calixarene and Cucurbituril Macrocycles", JOURNAL OF THE AMERICAN CHEMICAL SOCIETY, vol. 131, no. 32, August 2009 (2009-08), pages 11558-11570, ISSN: 0002-7863
- WONGKONGKATEP JIRARUT ET AL: "Label-free, real-time glycosyltransferase assay based on a fluorescent artificial chemosensor.", ANGEWANDTE CHEMIE (INTERNATIONAL ED. IN ENGLISH) 16 JAN 2006, vol. 45, no. 4, 16 January 2006 (2006-01-16), pages 665-668, ISSN: 1433-7851
- BAILEY DAVID M. ET AL: 'Supramolecular tandem enzyme assays for multiparameter sensor arrays and enantiomeric excess determination of amino acids.' CHEMISTRY (WEINHEIM AN DER BERGSTRASSE, GERMANY) 2008 Bd. 14, Nr. 20, 2008, Seiten 6069 - 6077 ISSN: 0947-6539
- NAU WERNER M. ET AL: 'Substrate-Selective Supramolecular Tandem Assays: Monitoring Enzyme Inhibition of Arginase and Diamine Oxidase by Fluorescent Dye Displacement from Calixarene and Cucurbituril Macrocycles' JOURNAL OF THE AMERICAN CHEMICAL SOCIETY Bd. 131, Nr. 32, August 2009, Seiten 11558 - 11570 ISSN: 0002-7863
- WONGKONGKATEP JIRARUT ET AL: 'Label-free, real-time glycosyltransferase assay based on a fluorescent artificial chemosensor.' ANGEWANDTE CHEMIE (INTERNATIONAL ED. IN ENGLISH) 16 JAN 2006 Bd. 45, Nr. 4, 16 Januar 2006, Seiten 665 - 668 ISSN: 1433-7851

## Beschreibung

Die Erfindung betrifft das Gebiet der makrozyklischen Wirtsysteme und Fluoreszenzfarbstoffe. Im Besonderen betrifft die Erfindung Vorrichtungen und Verfahren zum Bestimmen einer Konzentrationsänderung eines Analyten infolge einer katalysierten Reaktion, vorzugsweise einer enzymatisch katalysierten Reaktion und bevorzugt in wässriger (> 50 Gew.-% Wassergehalt) Lösung.

Zur Charakterisierung biochemischer Reaktionen ist es häufig notwendig, Konzentrationsänderungen von Reaktionspartnern zu bestimmen, insbesondere von Reaktionsprodukten und/oder -edukten. üblicherweise wird dazu ein Reaktionspartner als Analyt ausgewählt, dessen Konzentrationsänderung während des Ablaufs der zu charakterisierenden Reaktion bestimmt wird. üblicherweise wird die Anaiyt-Konzentration bestimmt durch (a) die Messung einer Substanzeigenschaft, die für den Analyten in der jeweiligen Reaktion spezifisch ist, oder (b) durch die Umsetzung des Analyten mit einer weiteren Substanz, wobei eine nachweisbare spezifische Stoffänderung der weiteren Substanz erreicht wird. Beispielsweise kann die Konzentration eines Analyten auf einem Weg der Art (a) anhand seiner Fluoreszenz bestimmt werden. Nachteilig hieran ist, dass nur wenige Substanzen geeignet für einen fluoreszenzspektroskopischen Nachweis sind, so dass dieses Verfahren nur für wenige Reaktionen durchführbar ist. Üblicherweise wird deshalb die Konzentration eines Analyten auf einem Weg der Art (b) bestimmt, beispielsweise durch Binden des Analyten an einen Antikörper und nach Bestimmen des Anteils derjenigen Antikörper, die den Analyten gebunden haben, im Vergleich zur Gesamtmenge an zur Verfügung stehenden Antikörpern. Ein solcher Nachweis kann beispielsweise über das Biacore-System erfolgen. Dabei hat es sich als nachteilig herausgestellt, dass bei rasch schwankender Analyt-Konzentration die Analyten nicht oder nicht schnell genug von den sie bindenden Antikörpern gebunden oder freigesetzt werden können. Dies erfordert im allgemeinen Wartezeiten von ca. 20 Minuten (sogenannte Inkubationszeiten), die eine wiederholte Messung in kurzen Abständen bzw. eine kontinuierliche Messung routinemässig verhindern (siehe z.B. Geymayer, P., Bahr, N. & Reymond, J.-L. Chem. Eur. J. 5, 1006-1012 (1999)).

Zum Charakterisieren biochemischer Reaktionen wird deshalb allgemein versucht, die Konzentration des zu analysierenden Analyten unmittelbar, ohne Vermittlung weiterer für den Nachweis erforderlicher Substanzen und ohne chemische Veränderung des Analyten, zu bestimmen. Nachteilig hieran ist, dass für viele Analyten ein jeweils spezielles Analyseverfahren entwickelt werden muss, beispielsweise weil sich die chemische Struktur der Analyten und dementsprechend ihre chemischen und physikalischen Eigenschaften voneinander unterscheiden.

Es ist deshalb versucht worden, nachzuweisende Analyten durch Versehen mit einer nachweisbaren Gruppe, beispielsweise eines Fluorophors, leichter nachweisbar zu machen. Dazu wird der Analyt vor Durchführen der Reaktion kovalent mit einer fluoreszierenden Gruppe verbunden. Bei Durchführen der Reaktion und entsprechender Umsetzung des Analyten soll die Fluoreszenz der fluoreszierenden Gruppe geändert werden, beispielsweise durch Abspaltung der Gruppe oder durch Änderung der Struktur des Analyten. Die Fluoreszenzänderung ist dann ein Maß für die Umsetzung des Analyten. Nachteilig hieran ist jedoch, dass die fluoreszierende Gruppe die Reaktionsfähigkeit des Analyten häufig beeinflusst. Insbesondere bei enzymatisch katalysierten Reaktionen ist es möglich, dass die fluoreszierende Gruppe die Bindung des Analyten an oder das Ablösen des Analyten vom Enzym verhindert oder erschwert, so dass die Charakterisierung der zu charakterisierenden Reaktion bzw. des anhand der Reaktion zu charakterisierenden Enzyms fehlerbehaftet ist. Nachteilig ist ferner der mit der notwendigen kovalenten Verknüpfung des Analyten mit der fluoreszierenden Gruppe verbundene Arbeitsaufwand.

In der Schrift von Wiskur et al. (Teaching old indicators new tricks, ACC. Chem. Res. 2001, 34, 963-972**)** wird beschrieben, dass die meisten synthetischen Sensoren dergestalt ausgelegt werden, dass ein kovalentes Anheften zwischen dem Rezeptor und einem Restreporter erfolgt. Weiterhin wird dort beschrieben, wie nicht-kovalente angebundene Indikatoren zur Signalbildung bei Analyten dienen.

EP 0010615 A1 (BOEHRINGER MANNHEIM GMBH [DE]) offenbart zwei Arten von Systemen zum Nachweis eines Analyten. Im ersten System ist ein Chromophor an ein Wirtsmolekül (oder Komplexligand), kovalent oder nicht-kovalent, verbunden. Durch Interaktion zwischen Analyt und Chromophor / Wirtsmolekül (bzw. Chromophor / Komplexligand) resultiert eine Extinktionsänderung oder Wellenlängenverschiebung des Chromophors, ohne das dieser vom Wirtsmolekül / Komplexligand verdrängt wird. Im zweiten System ist der Chromophor, wie in der vorliegenden Anmeldung, im Wirtsmolekül (bzw. Komplexligand) eingelagert und wird durch den Analyten kompetitiv verdrängt, was wiederum durch die resultierende Extinktionsänderung (oder Wellenlängenverschiebung) des Chromophors nachweisbar ist. Die Anmeldung erwähnt, dass die offenbarte Erfindung bei der kinetischen Verfolgung enzymatischer Prozesse Anwendung findet. Dies bezieht sich allerdings ausdrücklich nur auf das erste System.

Es war deshalb eine Aufgabe der vorliegenden Erfindung, ein Verfahren zum Bestimmen einer Konzentrationsänderung eines Analyten in einer Probe anzugeben, das die obigen Nachteile vermeidet oder verringert. Das Verfahren sollte insbesondere für eine Vielzahl von Analyten anwendbar sein, es sollte einfach durchführbar sein, es sollte die Bestimmung von sowohl langsamen (k_{cat} im Bereich von 5 bis 10⁴s⁻¹, vorzugsweise im Bereich von 5 bis 15 s⁻¹), als auch raschen (k_{cat} größer als 10⁴ s⁻¹, vorzugsweise im Bereich von 10⁵ bis 10⁷ s⁻¹ und besonders bevorzugt im Bereich von 5*10⁵ bis 5*10⁸ s⁻¹) Konzentrationsänderungen ermöglichen, anstatt lediglich die Bestimmung von Analyt-Konzentrationen in Reaktionsgleichgewichten zu ermöglichen, das Verfahren sollte eine online-Verfolgung des Reaktiönsverlaufs ohne merkliche Zeitverzögerung ermöglichen, es sollte mit der Möglichkeit, aber ohne die Notwendigkeit zur Probenahme aus der ablaufenden Reaktion durchführbar sein und/oder leicht adaptierbar sein für ein Hochdurchsatz-Analyseverfahren.

Eine weitere Aufgabe war es, eine Vorrichtung anzugeben, mit der das erfindungsgemäße Verfahren ausgeführt werden kann. Die Vorrichtung sollte insbesondere leicht zu bedienen sein, eine rasche Bestimmung von Analyt-Konzentrationsänderungen ermöglichen eine online-Bestimmung der Analyt-Konzentrationsänderung ermöglichen und/oder zur Durchführung von Hochdurchsatz-Testverfahren geeignet sein und dazu die rasche Bestimmung zahlreicher Proben ermöglichen.

Erfindungsgemäß wird entsprechend ein Verfahren zum Bestimmen einer Konzentrationsänderung eines Analyten in Folge einer katalytischen Reaktion in einer Probe angegeben, umfassend die Schritte:
a) Bereitstellen eines Fluoreszenzfarbstoffs und eines Makrozyklus in der zu untersuchenden Probe, wobei der Makrozyklus den Farbstoff binden und der Analyt den Fluoreszenzfarbstoff in dem zu untersuchenden Konzentrationsbereich des Analyten vom Makrozyklus verdrängen kann, und
b) Messen einer Fluoreszenzeigenschaft des Fluoreszenzfarbstoffs an zumindest zwei Zeitpunkten.
ferner umfassend einen Reaktionspartner zum Umsetzen des Analyten, so dass der umgesetzte Analyt in dem zu untersuchenden Analyten-Konzentrationsbereich den Fluoreszenzfarbstoff nicht oder nicht so stark wie der Analyt vom Makrozyklus verdrängt, und/oder einen Vorläufer (Edukt) des Analyten, der zum Analyten umgesetzt wird, und der in dem zu untersuchenden Analyten-Konzentrationsbereich den Fluoreszenzfarbstoff nicht oder nicht so stark wie der Analyt vom Makrozyklus verdrängt.

Die Erfindung ermöglicht es somit auf besonders vorteilhafte Weise, ohne chemische Veränderung des Analyten und insbesondere ohne kovalentes Anbringen einer fluoreszenten Gruppe, die Änderung der Konzentration eines Analyten in einer Probe während der Durchführung einer katalytischen Reaktion zu bestimmen. Dabei entkoppelt das erfindungsgemäße Verfahren auf vorteilhafte und überraschende Weise die an der zu charakterisierenden Reaktion beteiligten Substanzen von den zum Nachweis der Analyt-Konzentrationsänderung verwendeten Substanzen. Die Bestimmung der Analyt-Konzentrationsänderung wird somit ermöglicht, ohne dass ein Analyt eine dauerhafte oder die Reaktlonskinetik der zu untersuchenden Reaktion beeinflussende kovalente Bindung mit einer weiteren Substanz eingehen müsste. Damit verkleinert das erfindungsgemäße Verfahren auf überraschend einfache und vorteilhafte Weise eine mögliche Fehlerquelle, die ansonsten das Charakterisieren einer Reaktion und die Bestimmung einer Analyt-Konzentrationsänderung verfälschen oder beeinträchtigen könnte. Dabei ist es zweckmäßig dass, wenn die Analytkonzentration sich infolge einer chemischen katalytischen Umsetzung ändert, ein entsprechendes Umsetzungsprodukt des Analyten bzw. eine entsprechende Vorstufe (Edukt) des Analyten den Fluoreszenzfarbstoff nicht oder nicht so stark wie der Analyt vom Makrozyklus verdrängt bzw. verdrängen kann. Für alle Aspekte der Erfindung ist es entsprechend hotwendig dass die Vorstufe (Edukt) des Analyten bzw. das Umsetzungsprodukt des Analyten eine geringere Bindungskonstante zum Makrozyklus aufweist als der Analyt; vorzugsweise ist diese Bindungskonstante um mindestens einen Faktor 1,5 geringer als die des Analyten, und besonders bevorzugt um einen Faktor von 10-1000 geringer. Die Bindungskonstante kann insbesondere bestimmt werden wie in Adv. Funct. Mater. 2006, 16, 237-242 beschrieben.

Die zu den zwei Zeitpunkten gemessenen Werte der jeweiligen Fluoreszenzeigenschaft(en) werden vorzugsweise zum Berechnen einer für den Unterschied der Fluoreszenzeigenschaft repräsentativen Größe verwendet. Bevorzugt zu messende Fluoreszenzeigenschaften sind: Fluoreszenzintensität als besonders bevorzugte Fluoreszenzeigenschaft, sowie Fluoreszenzlebensdauer, Fluoreszenzpolarisation, Fluoreszenzspektrum, und Absorption bei einer vorgewählten Wellenlänge. Eine Fluoreszenzlebensdauer wird vorzugsweise bestimmt wie in Kapitel 4 und 5 von Lakowicz, J. R., Principles of Fluorscence Spectroscopy. Kluwer Academic/Plenum: New York, 1999 beschrieben. Es können auch zwei oder mehr der genannten Eigenschaften gemessen und zum Bestimmen einer Konzentrationsänderung des Analyten ausgewertet werden.

Vorzugsweise wird die Differenz oder das Verhältnis zwischen dem Wert der Fluoreszenzeigenschaft zum ersten und zweiten Messzeitpunkt bestimmt, beispielsweise die Differenz der ersten und zweiten Fluoreszenzintensität oder das Verhältnis aus zweiter zu erster Fluoreszenzintensität, wobei jedoch auch beliebige andere Verfahren zum Berechnen einer repräsentativen Größe gemeint sind.

Im Sinne der vorliegenden Erfindung ist ein Fluoreszenzfarbstoff ein Farbmittel, das ultraviolette Strahlung oder sichtbares Licht absorbieren und als Licht größerer Wellenlänge praktisch ohne zeitliche Verzögerung (fluoreszent) aussenden kann. Fluoreszenzfarbstoffe im Sinne dieser Erfindung sind sowohl organische, heteroorganische oder metallorganische Farbstoffmoleküle als auch chromophore Bestandteile (Fluorochrome) größerer molekularer Einheiten als auch lumineszierende Lanthanid-lonen; eine gute Übersicht über gängige Fluoreszenzfarbstoffe und ihre Einsatzgebiete ist erfindungsgemäß entnehmbar dem Handbook of Fluorescent Probes and Research Chemicals, Richard P. Haugland, Molecular Probes (Invitrogen).

Ein Makrozyklus im Sinne der Erfindung ist eine ringförmige Verbindung, die den Fluoreszenzfarbstoff unter Veränderung von dessen Fluoreszenzeigenschaften in einem inneren Hohlraum komplexieren kann. Bevorzugte Makrozyklen im Sinne dieser Erfindung sind Calixarene, Cucurbiturile und Cyklodextrine. Besonders bevorzugte Makrozyklen, insbesondere bevorzugte Calixarene, Cucurbiturile und Cyklodextrine, werden im weiteren Verlauf der Beschreibung beschrieben.

Ein Analyt im Sinne dieser Erfindung ist eine Substanz, die als Reaktionspartner an der zu untersuchenden Reaktion teilnimmt. Der Analyt kann insbesondere ein Reaktionsprodukt oder ein Reaktionsedukt sein. Der Analyt wird im Laufe der zu untersuchenden Reaktion in seiner chemischen Struktur verändert. In bevorzugten Ausführungsformen der Erfindung wird die Ladung, die Konstitution, oder Konfiguration des Analyten geändert. Der Analyt ist vorzugsweise eine organische oder heteroorganische Verbindung. Der Analyt besteht vorzugsweise aus oder umfasst kovalent miteinander verbundene Elemente ausgewählt aus der Gruppe bestehend aus Kohlenstoff, Sauerstoff, Stickstoff, Phosphor, Schwefel und Wasserstoff.

Erfindungsgemäß sind der Fluoreszenzfarbstoff und der Makrozyklus auf den Analyten abgestimmt, dessen Konzentrationsänderung bestimmt werden soll. Der Fluoreszenzfarbstoff wird vom Makrozyklus nach Art einer Wirts-Gast-Beziehung komplexiert, wobei die Fluoreszenzeigenschaften, insbesondere eine oder mehrere der oben aufgezählten Fluoreszenzeigenschaften, des komplexierten Fluoreszenzfarbstoffs gegenüber seinen entsprechenden Fluoreszenzeigenschaften in freier Lösung verändert sind. Der Analyt kann den Fluoreszenzfarbstoff vom Makrozyklus verdrängen. Je nach Analyt-Konzentration wird deshalb eine unterschiedlich große Menge an Fluoreszenzfarbstoff von den Makrozyklus-Molekülen freigesetzt. Damit ist die Fluoreszenz des Fluoreszenzfarbstoffs in dem zu untersuchenden Konzentrationsbereich abhängig von der Konzentration des Analyten, ohne dass der Fluoreszenzfarbstoff mit dem Analyten kovalent verbunden wäre oder eine andere Verbindung des Analyten mit dem Fluoreszenzfarbstoff eingegangen werden müsste. In besonders bevorzugten Ausführungsformen der Erfindung sind dementsprechend der Analyt, der Fluoreszenzfarbstoff und der Makrozyklus jeweils einzelne Moleküle, die miteinander nicht kovalent verbunden sind.

Aus dem Stand der Technik bekannt waren bisher lediglich Verfahren zum Nachweisen anorganischer oder organischer Kationen mit p-Sulfonatocalix[4]aren durch Verdrängung eines fluoreszenten Azoalkans vom Calixaren (Bakirci et al., Chem., Commun., 2005, 5411 bis 5413, sowie Adv. Funct. Mater. 2006, 16, 237-242). Die US 6,475,803 B1 zeigt ferner ein Verfahren zum Überprüfen, ob zumindest eine beliebige aus sieben organischen Verbindungen in Trinkwasser enthalten ist, wobei kovalent eine fluoreszente Gruppe an ein Cyclodextrin angebracht ist. Beide Verfahren zeigen lediglich die Möglichkeit, eine vorgegebene Konzentration zu bestimmen. Sie zeigen hingegen nicht, dass es möglich wäre, eine Konzentrationsänderung eines Analyten während einer Reaktion und insbesondere einer katalysierten und insbesondere einer enzymatisch katalysierten Reaktion zu bestimmen. Insbesondere geht aus den genannten Veröffentlichungen nicht hervor, mit welcher Geschwindigkeit die Änderung der Fluoreszenz eine Konzentrationsänderung des Kations nachvollziehen könnte.

Erfindungsgemäß wurde nunmehr erstmals erkannt, dass eine starke chemische Wechselwirkung und insbesondere eine kovalente Verbindung zwischen dem Analyten, dessen Konzentrationsänderung bestimmt werden soll, und einer die Konzentrationsänderung anzeigenden Substanz nicht notwendig ist, stattdessen wird im erfindungsgemäßen Verfahren auf überraschend schnelle Weise eine Konzentrationsänderung durch Vermittlung eines Makrozyklus nachweisbar gemacht, indem ein Analyt den Fluoreszenzfarbstoff vom Makrozyklus verdrängt. Das erfindungsgemäße Verfahren ermöglicht so erstmals eine spezifische online-Überwachung einer ablaufenden Reaktion ohne merkliche Verzögerung für eine Vielzahl von verschiedenen Analyten, ohne dass zuvor eine aufwendige Synthese von Fluorophor-Analyt-Fusionsmolekülen notwendig wäre.

Die Konzentrationsänderung des Analyten kann beispielsweise nachgewiesen werden, wenn der Analyt ein Reaktionsedukt ist, das im Verlauf der Reaktion zu einem oder mehreren Produkten umgesetzt wird, die den Fluoreszenzfarbstoff nicht mehr vom Makrozyklus verdrängen können. In diesem Fall ist die Konzentration des Fluoreszenzfarbstoffs in freier Lösung zu Beginn der Reaktion vergleichsweise hoch, da der Analyt den Fluoreszenzfarbstoff weitgehend oder vollständig von den Makrozyklus-Molekülen verdrängt. Im Verlauf der Reaktion nimmt die Analyt-Konzentration ab, so dass ein größerer Anteil an Fluoreszenzfarbstoff-Molekülen mit den Makrozyklus-Molekülen komplexieren kann. Dementsprechend ändert sich die Fluoreszenz einer zu untersuchenden Probe, in der die zu untersuchende Reaktion abläuft.

Wenn der Analyt ein Reaktionsprodukt ist, dann ist zu Beginn der Reaktion ein vergleichsweise hoher Anteil an Fluoreszenzfarbstoff-Molekülen mit Makrozyklus-Molekülen komplexiert. Im Verlaufe der Reaktion wird ein zunehmend größerer Anteil an Fluoreszenzfarbstoff-Molekülen von den Makrozyklus-Molekülen verdrängt, so dass sich die Fluoreszenz einer Probe, in der die zu untersuchende Reaktion stattfindet, dementsprechend ändert.

Zur Auswahl von Fluoreszenzfarbstoffen und Makrozyklen kann sich der Fachmann insbesondere einiger Leitlinien bedienen:

Wenn der Analyt, dessen Konzentrationsänderung bestimmt werden soll, ein Anion ist, dann ist der Makrozyklus zweckmäßigerweise ein Anionen-Rezeptor. Vorzugsweise ist der Makrozyklus für anionische Analyten ein Calixaren oder Cyclophan mit kationischen Gruppen. Ein Makrozyklus, der Anionen komplexieren kann, und insbesondere ein Calixaren mit kationischen Gruppen ist entsprechend vorteilhaft geeignet zur Verwendung als Makrozyklus in einer Reaktion, bei der (a) ein Anion in ein ungeladenes Molekül oder ein ungeladenes Molekül in ein Anion umgewandelt wird und (b) ein Anion in ein anderes Anion mit einer unterschiedlichen Ladung oder einer unterschiedlichen Größe oder einer unterschiedlichen Form (Konstitution oder Konfiguration) umgewandelt wird. Wenn der Analyt ein Anion ist, dann ist vorzugsweise auch der Fluoreszenzfarbstoff ein Anion.

Wenn der Analyt, dessen Konzentrationsänderung bestimmt werden soll, ein Kation ist, dann ist der Makrozyklus zweckmäßigerweise ein Kationen-Rezeptor. Vorzugsweise ist der Makrozyklus für kationische Analyten ein Calixaren oder Cyclophan mit anionischen Gruppen oder ein Cucurbituril. Ein Makrozyklus, der Kationen komplexieren kann, und insbesondere ein Cucurbituril oder ein Calixaren oder Cyclophan mit anionischen Gruppen ist entsprechend vorteilhaft geeignet zur Verwendung als Makrozyklus in einer Reaktion, bei der (a) ein Kation in ein ungeladenes Molekül oder ein ungeladenes Molekül in ein Kation umgewandelt wird und (b) ein Kation in ein anderes Kation mit einer unterschiedlichen Ladung oder einer unterschiedlichen Größe oder einer unterschiedlichen Form (Konstitution oder Konfiguration) umgewandelt wird. Wenn der Analyt ein Kation ist, dann ist vorzugsweise auch der Fluoreszenzfarbstoff ein Kation.

Wenn der Analyt eine Wasserstoffbrückenbindung eingehen kann, dann ist der Makrozyklus vorzugsweise ein Makrozyklus mit der Fähigkeit zur Ausbildung von Wasserstoffbrückenbindungen. Bevorzugte Makrozyklen mit der Fähigkeit zum Ausbilden von Wasserstoffbrückenbindungen sind Cyclodextrine. Makrozyklen mit der Fähigkeit zum Ausbilden von Wasserstoffbrückenbindungen und insbesondere Cyclodextrine sind entsprechend vorteilhaft verwendbar zum Bestimmen einer Konzentrationsänderung eines Analyten in einer Reaktion, bei der (a) eine nicht zur Bildung von Wasserstoffbrückenbindungen fähige Substanz in einen Wasserstoffbrücken-Donor oder -Akzeptor als Analyt umgewandelt wird oder umgekehrt, (b) bei der ein Wasserstoffbrücken-Donor in einen Wasserstoffbrücken-Akzeptor umgewandelt wird, wobei der Wasserstoffbrücken-Donor oder der Wasserstoffbrücken-Akzeptor der Analyt sein kann, oder (c) ein zum Bilden von Wasserstoffbrücken fähiger Analyt in eine Substanz mit anderer Größe oder einer unterschiedlichen Form (Konstitution oder Konfiguration) umgewandelt wird. Vorzugsweise ist der Fluoreszenzfarbstoff ebenfalls eine zum Ausbilden von Wasserstoffbrückenbindungen als Donor bzw. Akzeptor fähige Substanz.

Falls der Analyt lediglich durch seine Größe von anderen an der zu untersuchenden Reaktion beteiligten Substanzen unterschieden ist, dann wird der Makrozyklus vorzugsweise so ausgewählt, dass er Substanzen von der Größe des Analyten, nicht aber von der Größe eines Umsetzungsproduktes des Analyten bzw. einer in den Analyten umzuwandelnden Vorstufe, komplexieren kann. Der Makrozyklus kann dabei vorzugsweise ein Calixaren, ein Cyclophan ein Cucurbituril oder ein Cyclodextrin sein. Der Fluoreszenzfarbstoff besitzt dann zweckmäßigerweise eine ähnliche Größe wie der mit dem Makrozyklus komplexierende Analyt.

Falls der Analyt stereoselektiv von einer Vorform (Edukt) oder einem Reaktionsprodukt unterschieden werden soll, ist der Makrozyklus vorzugsweise ein Cyclodextrin mit geeigneter Größe zum Binden des Analyten.

Bevorzugte Calixaren-Makrozyklen sind: 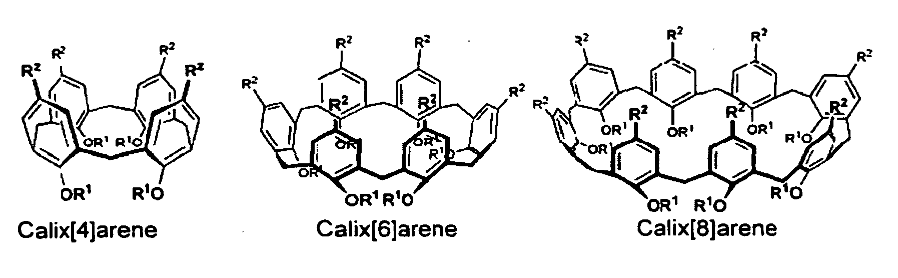 wobei, unabhängig voneinander,
R¹ = H, (CH₂)ₘCH₃, wobei m = 0, 1, 2, 3, 4, 5, 6 oder 7 oder ein verzweigtes oder geradkettiges C₁-C₆-Alkyl oder C₁-C₈-Alkenyl, vorzugsweise iso-Propyl, tert-Butyl;
R² = (CH₂)ₘX, wobei m = 0,1,2 und X = F, Cl, Br, I, SO₃H, SH, OH, COOH, NH₂, NRH, NR₂, NR₃⁺ (jeweils mit R ausgewählt aus Methyl, Ethyl, n-Propyl und iso-Propyl).

Ein bevorzugter Cyclophan-Makrozyklus ist: 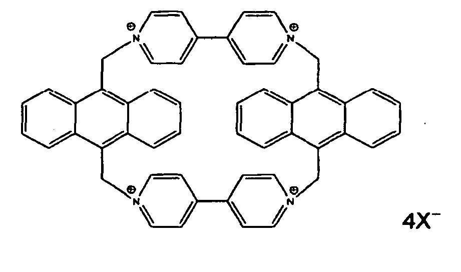 wobei X- ein Gegenanion ist, bevorzugt Chlorid.

Bevorzugte Cyclodextrin-Makrozyklen sind: 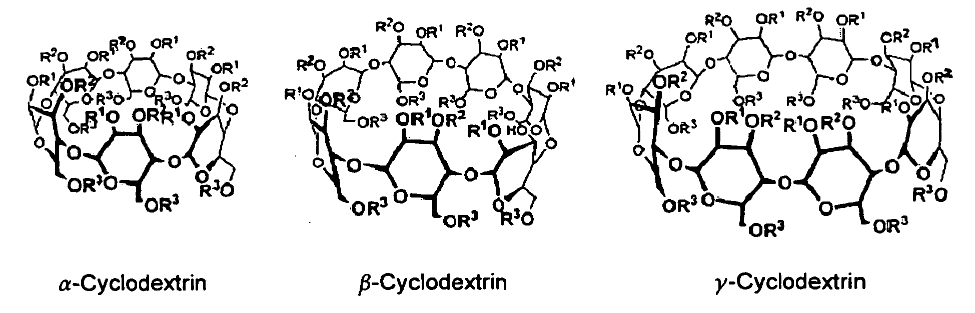 wobei, unabhängig voneinander, jedes R¹, R², R³ ausgewählt ist aus H, Methyl, Ethyl, n-Propyl, iso-Propyl, Butyl, Octyl, (CH2)_{1,2}COOH, (CH2)₂,₃OH, Acetyl, Benzoyl, Sulfo, Maltosyl, Hydroxypropyl, Succinyl und anstelle von OR³ auch eine Gruppe NHR⁴, NR⁴R⁵ und/oder (NR⁴R⁵R⁶)⁺ vorgesehen sein kann, wobei R⁴, R⁵ und R⁶ wiederum, unabhängig voneinander, ausgewählt sind aus H, Methyl, Ethyl, n-Propyl, iso-Propyl, Butyl, Octyl, (CH2)_{1,2}COOH, (CH2)_{2,3}OH, Acetyl, Benzoyl, Sulfo, Maltosyl, Hydroxypropyl, Succinyl.

Bevorzugte Cucurbituril-Makrozyklen sind: 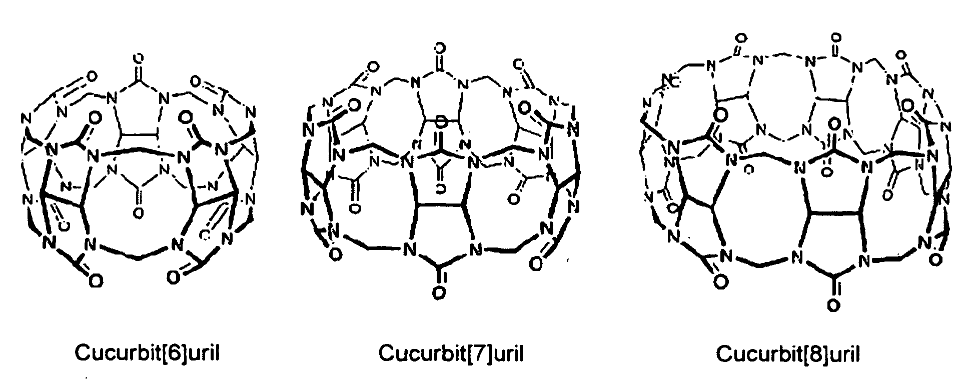 und allgemein 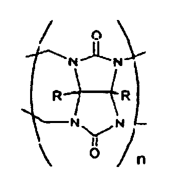 wobei n = 6,7,8 und jedes R, unabhängig von jedem anderen R, ausgewählt ist aus H, Methyl und Hydroxy.

Geeignete und bevorzugte Makrozyklus-Fluoreszenzfarbstoff-Paare kann der Fachmann durch Anwendung eines der oben beschriebenen Auswahlverfahren zusammenstellen. Für eine Vielzahl von Analyten sind insbesondere die in der nachfolgenden Tabelle I angegebenen Makrozyklus-Fluoreszenzfarbstoff-Paare geeignet und bevorzugt:

**Tabelle I**

| Makrozyklus | Fluoreszenzfarbstoff |
|---|---|
| Calix[n]aren | DBO-Essigsäure |
| n=4 | |
| R¹ = H,Alkyl | |
| R² = (CH₂)ₘNR₂ | 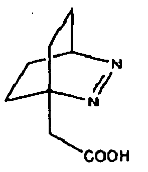 |
| wobei m = 0,1,2 | |
| und R = H, Me, Et | |
| Calix[n]aren | 1-Anilinonaphtalin-8-sulfonat(1,8-ANS) |
| n = 4,6,8 | 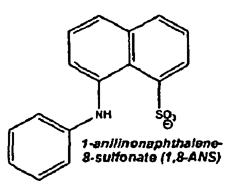 |
| R¹ = H,Alkyl | |
| R² = (CH₂)ₘNR₂ | |
| oder (CH₂)ₘNR₃⁺ | |
| wobei m = 0,1,2 | |
| und R = H, Me, Et, Allyl | |
| Calix[n]aren | DBO-Amin |
| n=4 | 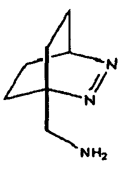 |
| R¹ = H,Alkyl | |
| R² = SO₃H | |
| Calix[n]aren | Acridin Rot (AR) |
| n = 4,6,8 | 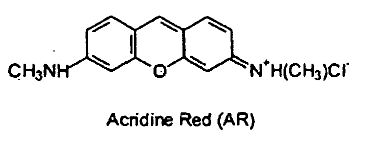 |
| R¹ = H,Alkyl | |
| R² = SO₃H | |
| Calix[n]aren | Stilben-Farbstoffe |
| n = 4,6,8 | 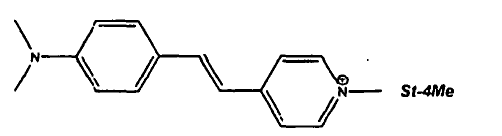 |
| R¹ = H,Alkyl | |
| R² = SO₃H | |
| | 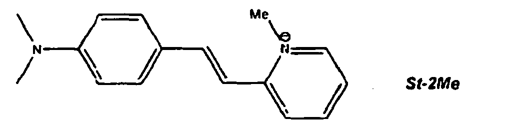 |
| | 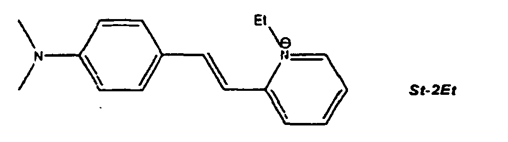 |
| | Diese Farbstoffe binden sehr gut an R¹ = H (auch an n = 4). |
| Calix[n]aren | Rhodamin B |
| n = 6,8 | 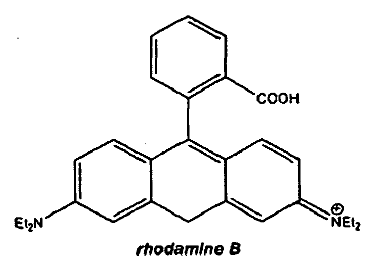 |
| R¹ = H,Alkyl | |
| R² = SO₃H | |
| Calix[n]aren | Auramin O |
| n = 4,6,8 | 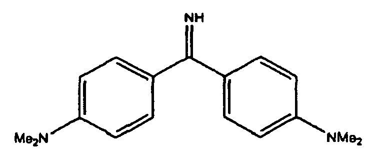 |
| R¹ = H,Alkyl | |
| R² = SO₃H | |
| Calix[n]arene | Acridin Orange (AO) |
| n = 4,6,8 | 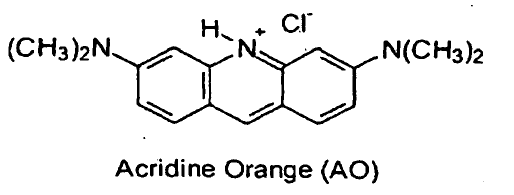 |
| R¹ = H,Alkyl | |
| R² = SO₃H | |
| Calix[n]arene | Pyronin B (R = Et), Pyronin Y (R = Me) |
| n = 6,8 | 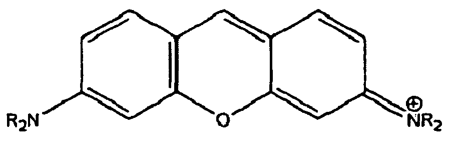 |
| R¹ = H,Alkyl | |
| R² = SO₃H | |
| Bevorzugte Bedingungen für die obigen Calixaren-Fluoreszenzfarbstoff-Paare: wässrige Lösung (auch gepuffert: z.B. Citrat, Acetat, Phosphat-Puffer), pH = 2 bis 9 | |
| Cyclophan xxx | 8-Hydroxy-1,3,6-pyrentrisulfonat |
| 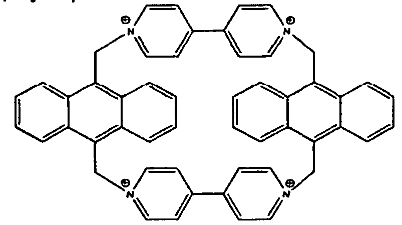 | 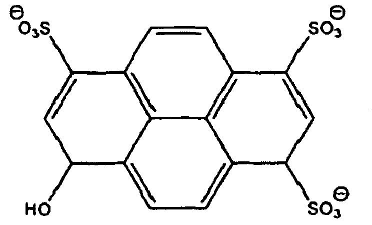 |
| Resorc[4]aren | Pyren- und Dansylderivat |
| 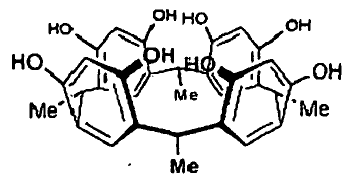 | 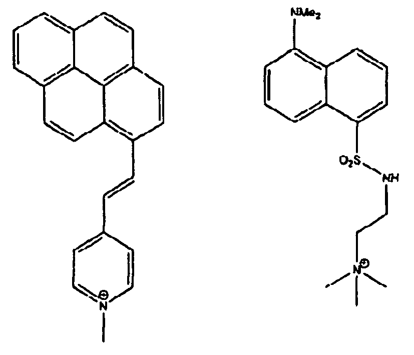 |
| Bevorzugte Bedingungen: wässrige Lösung (auch gepuffert: z.B. Citrat, Acetat, Phosphat-Puffer), pH = 2 bis 12 | |
| β-Cyclodextrin | DBO |
| R¹=R²=R³=H | 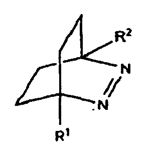 |
| | R¹ = R² = H; R¹ = R² = Cl, R¹ = Me, R² = i-Pr |
| β-Cyclodextrin | Acridin Rot (AR) |
| R¹ = R² = R³ = H | 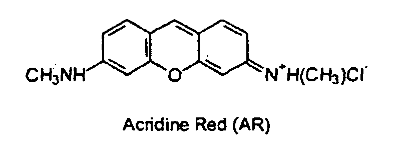 |
| β-Cyclodextrin | Natrium 6-Toluidino-2-Naphthalensulfonat (TNS) |
| R¹=R²=R³=H | 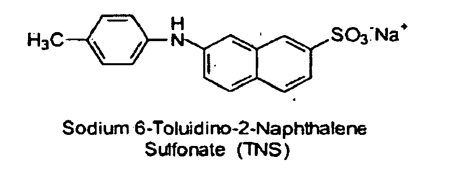 |
| β-Cyclodextrin | Crystal Violet (CV) |
| R¹=R²=R³=H | 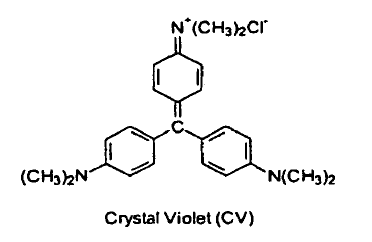 |
| β-Cyclodextrin | Phthalimide |
| R¹=R²=R³=H | 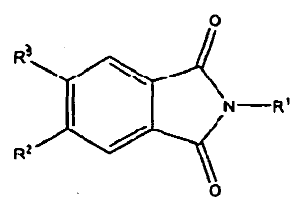 |
| | Vorzugsweise: |
| | R¹ = *t*-Bu, Cyclohexyl, Adamantyl |
| | R² = NH₂, R³ = H |
| β-Cyclodextrin | 2-anilinonaphtalin-8-sulphonat(2,6-ANS) |
| R¹ = R² = R³ = H,Me,Hydroxypropyl | 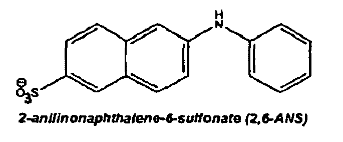 |
| β-Cyclodextrin | 1-anilinonaphtalin-8-sulphonat(1,8-ANS) |
| R¹ = R² = R³ = Me,Hydroxypropyl | 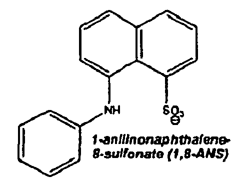 |
| γ-Cyclodextrin | |
| R¹ = R² = R³ = Hydroxypropyl | |
| α-Cyclodextrin | Dimethylaminobenzonitril |
| R¹ = R²= R³ = H | 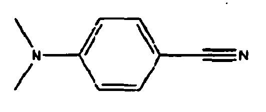 |
| β-Cyclodextrin | Perylium-Farbstoffe |
| R¹ = R² = R³ = H | 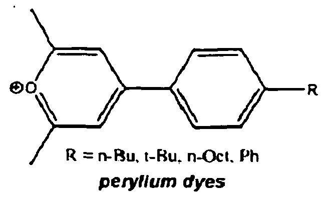 |
| | wobei R ausgewählt ist aus n-Butyl, t-Butyl, n-Octyl und Phenyl |
| Bevorzugte Bedingungen für Cyclodextrin-Fluoreszenzfarbstoff-Paare: wässrige Lösung (auch gepuffert: z.B. Citrat, Acetat, Phosphat-Puffer), pH = 2 bis 12 | |
| Cucurbiturile | Cucurbituril-Fluoreszenzfarbstoff-Paare sind angegeben in der WO2006/005727, deren Offenbarungsgehalt für die Zwecke dieser Erfindung vollständig in Bezug genommen wird |
| Cucurbit[7]uril | 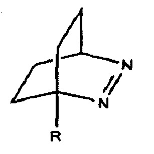 |
| | R = H, CH₂NH₂ |
| Cucurbit[7]uril | Dapoxyl |
| | 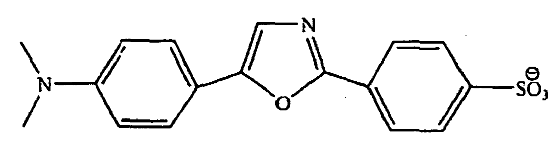 |
| Cucurbit[7]uril | Rhodamin 6G |
| Cucurbit[7]uril | Tetramethylrhodamin |
| Cucurbit[7]uril | Neutral Rot |
| | 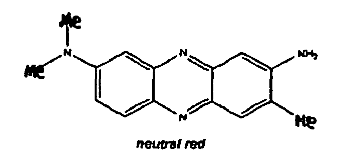 |
| Cucurbit[7]uril | 2-Aminoanthracen |
| Cucurbit[6]uril | Curcumin |
| | 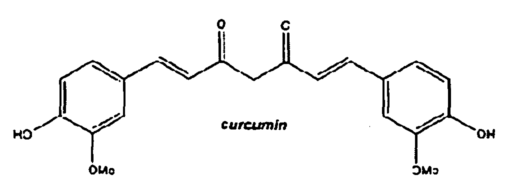 |
| Cucurbit[8]uril | 2-7-dimenthyldiazapyrenium |
| | 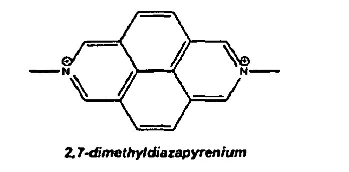 |
| 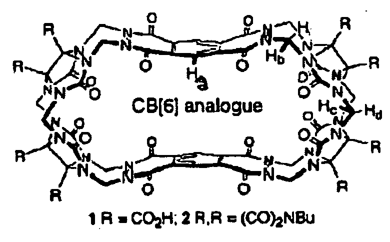 | Nile Rot und Nile Blau |
| | 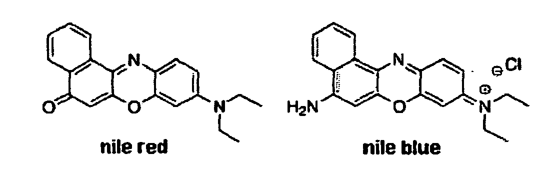 |
| Bevorzugte Bedingungen für Cucurbituril-Fluoreszenzfarbstoff-Paare: wässrige Lösung (auch gepuffert: z.B. Citrat, Acetat, Phosphat-Puffer), pH = 2 bis 12 | |

Die Analytkonzentration ändert sich vorzugsweise, weil der Analyt an einer Reaktion teilnimmt, in deren Verlauf er in eine andere Substanz umgewandelt wird, beispielsweise indem er isomerisiert wird, indem ihm eine oder mehr chemische Gruppen zugefügt oder von ihm abgenommen werden, oder indem seine Ladung geändert wird. Ebenfalls bevorzugt ist es, wenn der Analyt aus einer entsprechenden Reaktion hervorgeht, bei der eine Vorläufer-Substanz (Edukt) in den Analyten wie soeben beschrieben umgewandelt wird.

Bei dem erfindungsgemäßen Verfahren, kann in der Probe zusätzlich ein Reaktionspartner zum Umsetzen des Analyten oder zum Umsetzen einer Vorstufe (Edukt) zum Analyten vorliegen Derartige Reaktionen machen den Hauptteil der biochemisch relevanten Reaktionen aus. Das Durchführen eines erfindungsgemäßen Verfahrens, insbesondere mit dem zuvor angegebenen Leitlinien für die Makrozyklus- und Farbstoff-Auswahl, st dementsprechend erfindungsgemäß besonders bevorzugt.

Dabei ist es insbesondere bevorzugt, wenn der Reaktionspartner ein Katalysator und insbesondere ein Enzym ist. Erfindungsgemäß kann also der Reaktionspartner des Analyten bzw. der Analyten-Vorstufe aus der Reaktion unverändert hervorgehen, wie bei einem Katalysator oder Enzym üblich. Erfindungsgemäß bevorzugte Enzyme und Reaktionen sind in nachfolgender Tabelle II angegeben, wobei der Analyt jeweils ein Produkt oder Edukt der vom jeweiligen Enzym katalysierten Reaktion einschließlich eines Co-Faktors sein kann.

**Tabelle II**

| | |
|---|---|
| Enzymklasse | bevorzugte Unterklassen und bevorzugte Enzyme |
| EC 1 Oxidoreduktasen | alle ausgenommen EC 1.9.x.x. 1.15.x.x. 1.18.x.x, 1.19.x.x und 1.21.x.x, unberührt davon ist jedoch die Anwendung des Cofaktors als Analyt möglich, nämlich für die Klassen EC 1.x.1.x, 1.x.4.x, 1.x.5.x, 1.x.6.x, und teilweise für 1.x.98.x und 1.x.99.x |
| EC 2 Transferasen | alle |
| EC 3 Hydrolasen | alle |
| EC 4 Lyasen | alle |
| EC 5 Isomerasen | besonders bevorzugt: EC 5.1.x.x, EC 5.2.x.x und EC 5.4.x.x bis EC 5.99.x.x |
| EC 6 Ligasen | besonders bevorzugt EC 6.1.x.x, 6.2.x.x, 6.5.x.x und 6.6.x.x |

Das erfindungsgemäße Verfahren ermöglicht auf vorteilhaft einfache Weise das Charakterisieren von Enzymen und ermöglicht dabei jeweils eine weitgehend freie Wahl des Analyten. Auf diese Weise ermöglicht es das erfindungsgemäße Verfahren erstmals, die ansonsten nur durch gezielte Analyten-Auswahl oder durch die kovalente Verknüpfung eines Analyten mit einer nachweisbaren, beispielsweise fluorophoren, Gruppe mögliche und dementsprechend nur sehr aufwendige oder stark fehlerbehaftete Charakterisierung eines Enzyms.

Aufgrund seiner vielseitigen Verwendbarkeit kann das erfindungsgemäße Verfahren auf vorteilhaft einfache Weise aufgenommen werden in einem erfindungsgemäßen Screening-Verfahren. Erfindungsgemäß wird deshalb auch ein Screening-Verfahren zum Suchen eines Reaktionspartners für einen Analyten bereitgestellt, umfassend die Schritte:
a) Bereitstellen einer Anzahl von Mischungen, enthaltend jeweils den vorgewählten Analyten oder eine Vorstufe (Edukt) des Analyten, einen zu testenden Reaktionspartner zum Herstellen des Analyten aus der Vorstufe oder zum Umsetzen des Analyten, einen Fluoreszenzfarbstoff und einen Makrozyklus, wobei der Analyt den Fluoreszenzfarbstoff in den zu untersuchenden Konzentrationsbereich des Analyten vom Makrozyklus verdrängen kann, und optional einen Inhibitor der Reaktion, und
b) Durchführen eines erfindungsgemäßen Bestimmungsverfahrens mit jeder der in a) bereitgestellten Mischung.

Für jede der in a) bereitgestellten Mischung wird dementsprechend die Fluoreszenzeigenschaft des Fluoreszenzfarbstoffs, insbesondere die Fluoreszenzintensität, die Fluoreszenzlebensdauer, die Fluoreszenzpolarisation, das Fluoreszenzspektrum, und/oder die Absorption bei einer vorgewählten Wellenlänge, an zumindest zwei Zeitpunkten der in der Mischung ablaufenden Reaktion gemessen. Die Änderung der Fluoreszenzeigenschaft, beispielsweise der Fluoreszenzintensität, der jeweiligen Mischung ist dann ein Maß für die Änderung der Analyt-Konzentration. Durch Vergleich der Fluoreszenzänderungen verschiedener Mischungen können beispielsweise Analyt-Reaktionspartner identifiziert werden, die eine besonders schnelle Umsetzung des Analyten oder einer Vorstufe zum Analyten ermöglichen. Ein solches Verfahren ist deshalb besonders geeignet als Screening-Verfahren für Reaktionspartner einer enzymatisch katalysierten Reaktion. Beispielsweise kann in den zu untersuchenden Mischungen jeweils ein vorgewähltes Enzym und ein spezifischer Analyt bzw. eine Analyt-Vorstufe (also eine als Edukt zum Analyten umzusetzende Vorstufe) vorgelegt werden, wobei dann eine Substanz aus einer Substanz-Bibliothek zugegeben und die Änderung der Analyt-Konzentration bestimmt wird. Der Analyt eines solchen Screening-Verfahrens ist dann vorzugsweise ein Co-Faktor, insbesondere vorzugsweise Adenosintriphosphat, Adenosindiphosphat, Adenosinmonophosphat, Cycloadenosinmonophosphat, Nikotinsäureamid-Adenin-Dinukleotid (NAD+) und die entsprechende reduzierte Verbindung (NADH+H+), Nicotinamid-Adenin-Dinukleotid-Phosphat (NADP) und die entsprechende reduzierte Verbindung (NADPH+H+). Mit einem solchen Screening-Verfahren ist es auch möglich, einen Inhibitor für eine bekannte Reaktion zu suchen. Das systematische Suchen (Screenen) nach Inhibitoren für eine vorgewählte Reaktion ist wirtschaftlich besonders bedeutsam, insbesondere zur Suche nach Arzneimitteln. Dabei werden das oder die Edukt(e) der zu untersuchenden Reaktion vorgelegt und als Substanz aus der Substanz-Bibliothek (Inhibitor-Bibliothek) ein möglicher Inhibitor der Reaktion zugegeben. Die Fluoreszenzänderung als Maß der Konzentrationsänderung eines Analyten (der ein Reaktionsprodukt oder Edukt sein kann, insbesondere ein Co-Faktor wie soeben genannt) ist dann ein Maß für die inhibierende Wirkung der aus der Substanz-Bibliothek gewählten möglichen Inhibitor-Substanz. Wiederum ist es bevorzugt, einen Inhibitor für eine katalysierte und insbesondere enzymatisch katalysierte Reaktion zu suchen. Der Analyt kann dabei einer der vorhergenannten Co-Faktoren oder ein anderes Reaktionsprodukt oder Edukt sein. Insbesondere ist es mit dem erfindungsgemäßen Verfahren nunmehr möglich, Inhibitoren für eine Reaktion zu suchen, die durch eines der in Tabelle II genannten Enzyme katalysiert wird. Das erfindungsgemäße Verfahren eignet sich deshalb besonders für die Entwicklung von Arzneimitteln, indem es das Finden pharmazeutisch wertvoller Inhibitoren einer vorgewählten Reaktion in einer großen Bibliothek potentieller Inhibitoren (Inhibitor-Bibliothek) erleichtert oder erstmals praktisch ermöglicht. Besonders bevorzugt wird das Verfahren eingesetzt zum Finden von Inhibitoren für eine in wässriger Lösung durch eines der Enzyme der Tabelle II katalysierten Reaktion.

Das erfindungsgemäße Screening-Verfahren ist ferner geeignet zum Untersuchen, welcher Katalysator eine vorgegebene Reaktion katalysieren kann. Dazu werden das oder die Reaktionsedukt(e) vorgelegt und aus einer Substanz-Bibliothek (Katalysator-Bibliothek) ein jeweils möglicher Katalysator, insbesondere ein mögliches Enzym, beispielsweise aus einer Mutations-Bibliothek, ausgewählt und zugegeben. Die Fluoreszenzänderung ist dann ein Maß für die Änderung einer Analyt-Konzentration, wobei der Analyt wiederum ein Reaktionsprodukt oder Edukt sein kann, insbesondere einer der zuvor angegebenen Co-Faktoren oder ein anderes Reaktionsprodukt oder Edukt. Auf diese Weise kann beispielsweise untersucht werden, welches mögliche Enzym einer Enzym-Bibliothek eine vorgewählte Reaktion am schnellsten oder mit der besten Ausbeute katalysieren kann.

Zudem eignet sich das erfindungsgemäße Verfahren zum Bestimmen, welcher Katalysator einer Substanz-Bibliothek (Katalysator-Bibliothek) geeignet ist, eine vorgegebene Reaktion trotz Anwesenheit eines Inhibitors zu katalysieren. Dazu werden wiederum das oder die Reaktionsedukt(e) sowie der Inhibitor vorgelegt und aus einer Substanz-Bibliothek ein möglicher Katalysator, insbesondere ein Enzym einer Enzym-Mutanten-Bibliothek, zugegeben. Als Analyt dient wiederum ein Reaktionsprodukt oder Edukt, insbesondere einer der oben angegebenen Co-Faktoren. Die Fluoreszenzänderung als Maß der Konzentrationsänderung des Analyten zeigt dann die Fähigkeit und das Ausmaß der Fähigkeit des Katalysators und insbesondere des Enzyms an, die vorgewählte Reaktion trotz Anwesenheit des Inhibitors zu katalysieren.

Das erfindungsgemäße Verfahren, und insbesondere ein erfindungsgemäßes Screening-Verfahren, ist dementsprechend besonders vorteilhaft geeignet zum Suchen von pharmazeutisch wirksamen Bestandteilen, die beispielsweise eine vorgewählte Reaktion ermöglichen oder verhindern sollen. Das erfindungsgemäße Verfahren erfordert dabei nur eine minimale Anpassung an die jeweils zu untersuchende Reaktion, indem jeweils lediglich ein Makrozyklus-Fluoreszenzfarbstoff-Paar an den Analyten angepasst werden muss. Wenn als Analyt eine in mehreren biochemischen Reaktionen wiederkehrende Substanz, wie beispielsweise ein Enzym-Co-Faktor, und insbesondere einer der oben angegebenen Co-Faktoren, gewählt wird, kann ein standardisiertes erfindungsgemäßes Verfahren mit vorgewähltem Farbstoff und Makrozyklus eingesetzt werden. Das erfindungsgemäße Verfahren ist jedoch auch besonders vorteilhaft anwendbar zum Screenen einer Inhibitor-Bibliothek, also zum Untersuchen, welche Substanz eine vorgewählte Reaktion besonders gut inhibieren kann, und ebenfalls besonders gut geeignet zum Screenen einer Enzym-Bibliothek, also zum Untersuchen, welches Enzym bzw. welcher Katalysator einer Katalysator-Bibliothek eine vorgegebene Reaktion mit oder ohne Inhibitor besonders gut katalysieren kann. In diesen Fällen braucht lediglich einmal ein geeignetes Fluoreszenzfarbstoff-Makrozyklus-Paar ausgewählt zu werden und kann dann zum Screenen in einer Vielzahl von Mischungen verwendet werden.

Das erfindungsgemäße Verfahren, und insbesondere das erfindungsgemäße Screening-Verfahren, ist vorzugsweise ein Hochdurchsatz-Verfahren, und insbesondere ein Hochdurchsatz-Screening-Verfahren. Solche Hochdurchsatz-Verfahren (High Throughput-Verfahren) ermöglichen es, große Substanz-Bibliotheken in kurzer Zeit zu untersuchen, üblicherweise in einem standardisierten selbsttätig arbeitenden System, so dass Messfehler vorteilhaft klein gehalten werden können. Im Rahmen eines solchen Hochdurchsatz-Verfahrens, und insbesondere Hochdurchsatz-Screening-Verfahrens, werden vorzugsweise zumindest Vielfache von 48 Mischungen oder Proben, besonders vorzugsweise jedoch zumindest 96, 384 oder 1536 Mischungen oder Proben (gegebenenfalls einschließlich entsprechender Kontrollmischungen bzw. Kontrollproben) untersucht. Das erfindungsgemäße Verfahren ist besonders leicht automatisierbar und ermöglicht so das Untersuchen einer Substanz-Bibliothek, insbesondere einer Katalysator-, Enzym-, Inhibitor- und/oder sonstiger Reaktionspartner-Bibliotheken in kurzer Zeit mit geringem Messfehler.

Zum Durchführen des erfindungsgemäßen Verfahrens, insbesondere eines erfindungemäßen Screening-Verfahrens, wird eine Vorrichtung angegeben zum Bestimmen einer Konzentrationsänderung eines Analyten, umfassend
- eine Probe mit einem Fluoreszenzfarbstoff und einem Makrozyklus, wobei der Analyt den Fluoreszenzfarbstoff in dem zu untersuchenden Konzentrationsbereich des Analyten vom Makrozyklus verdrängen kann, und
- einen Reaktionspartner zum Umsetzen des Analyten.

Der Reaktionspartner kann dabei auch eine Vorstufe (Edukt) des Analyten zum Analyten umsetzen. Eine solche Vorrichtung, bei der ein Analyt stofflich verändert wird bzw. ein Analyt-Vorläufer stofflich zum Analyten verändert wird, ermöglicht auf besonders einfache Weise das Erreichen der mit einem erfindungsgemäßen Verfahren oben beschriebenen Vorteile. Es ist auch hier bevorzugt, wenn die Vorstufe des Analyten bzw. das Umsetzungsprodukt des Analyten den Fluoreszenzfarbstoff nicht oder nicht so stark wie der Analyt vom Makrozyklus verdrängen kann.

Die erfindungsgemäße Vorrichtung umfasst vorzugsweise eine Lichtquelle zum Anregen des Fluoreszenzfarbstoffs und einen Detektor zum Messen der vom Fluoreszenzfarbstoff ausgehenden Fluoreszenz und/oder Lichtabsorption. Ferner ist es bevorzugt, wenn die Vorrichtung einen Träger umfasst mit Aufnahmen für zumindest Vielfache von 48 zu untersuchende Proben oder Mischungen, vorzugsweise 96, 384 oder 1536 einschließlich gegebenenfalls vorgesehener Kontrollproben oder Kontrollmischungen. Besonders bevorzugte Träger sind so genannte Mikrotiter-Platten mit einer entsprechenden Anzahl an Kavitäten/Wells. Als wesentlicher Vorteil der erfindungsgemässen Methode gegenüber der Verwendung von Antikörpern kann die Verfolgung der Konzentrationsänderungen, insbesondere von enzymatisch bewirkten Konzentrationsänderungen, kontinuierlich erfolgen.

Die Erfindung wird nachfolgend anhand eines Ausführungsbeispiels und der Figuren weiter beschrieben, ohne dass dadurch der Schutzbereich der Patentansprüche eingeschränkt sein soll.

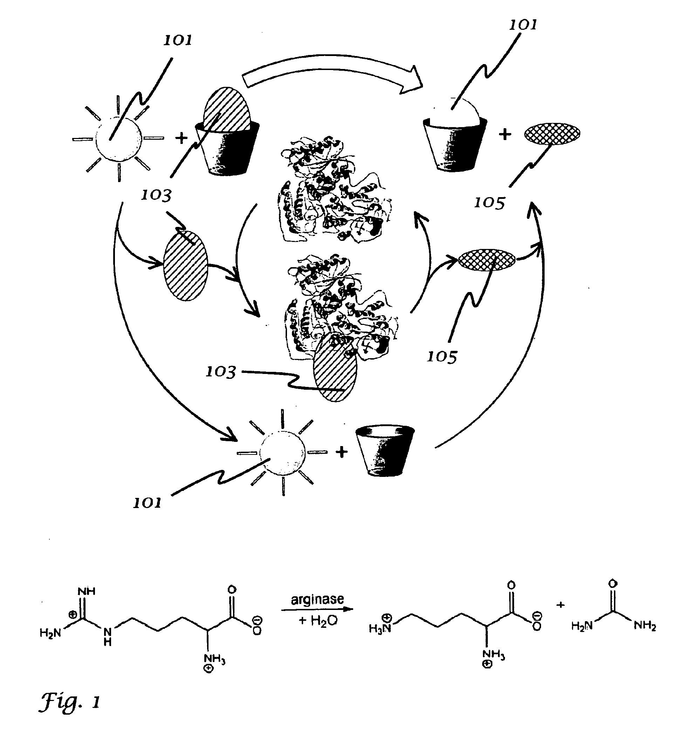

Figur 1 darstellend das Reaktionsschema der Umsetzung von L-Arginin **(103,** Analyt, verdrängt den Fluoreszenzfarbstoff) zu L-Ornithin **(105,** Produkt) und Harnstoff (nicht gezeigt, Produkt) durch L-Arginase. Als Fluoreszenzfarbstoff wurde 1-Aminomethyl-2,3-diazabicyclo[2.2.2]oct-2-en **(101,** DBO-Amin) gewählt und p-Sulfonato-Calix[4]aren (ein Kationenrezeptor) wurde als Makrozyklus verwendet, wobei die Fluoreszenz von DBO-Amin **101** durch das Calixaren gelöscht wird. Infolge des Abbaus des Analyten **103** wird eine Fluoreszenzabnahme während der enzymatischen Reaktion erwartet und beobachtet. Das Calixaren fungiert als grössenselektiver Rezeptor, der die grössere Guanidinium-Gruppe im Analyten Arginin **103** bei gleichbleibender positiver Ladung besser bindet als die kleinere Amino-Gruppe im Produkt Ornithin **101.** Die schematisierte Darstellung des Enzyms Arginase erfolgte in Anlehnung an die publizierte Kristallstruktur (Cama, E. et al., Biochemistry 43, 2004, 8987-8999. Protein Data Bank-ID: 1T4P) und wurde mit dem Programm Swiss-PdbViewer v3.7 (http://www.expasy.org/spdbv/) dargestellt.

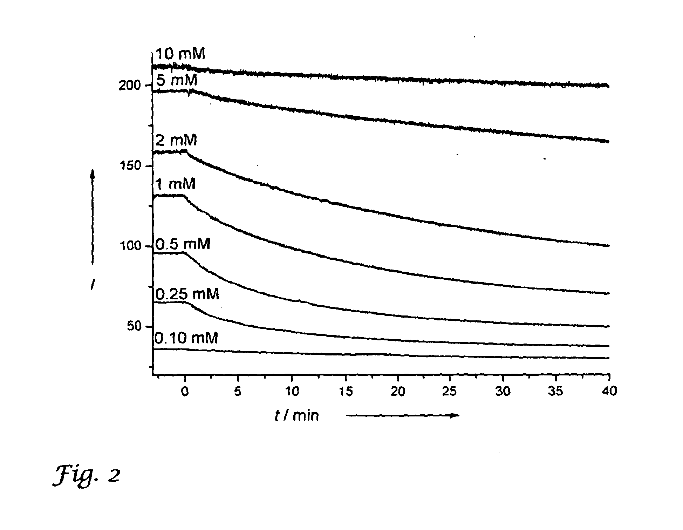

Figur 2 stellt die Fluoreszenzänderung nach Zugabe von 16 µg/ml Arginase (jeweils zum Zeitpunkt *t* = 0 Minuten) bei verschiedenen Konzentrationen von Arginin dar. Die enzymatische Reaktion kann somit in einfacher Weise ohne chemische Veränderung des *L-*Lysin, ohne radioaktive Markierung, und ohne Antikörper direkt in Lösung ohne weitere Inkubationsschritte mithilfe eines kommerziellen Fluoreszenzspektrometers (in diesem Fall: Varian Cary Eclipse, λ_{exc} = 365 nm, λ_{obs} = 450 nm) verfolgt werden. Eine deutliche Fluoreszenzänderung, gemessen als Verhältnis aus der Fluoreszenzintensität nach Beendigung der Reaktion und der Ausgangs-Fluoreszenzintensität ist bereits ab einer Arginin-Konzentrationen von 0,25 mM zu verzeichnen. Mit zunehmender Menge an Arginin nimmt die Ausgangsintensität zu, da mehr von dem Fluoreszenzfarbstoff aus dem die Fluoreszenz löschenden Makrozyklus verdrängt wird. Auch die Fluoreszenzintensität nach Beendigung der Reaktion nimmt mit zunehmender Argininausgangskonzentration zu, da das Produkt Ornithin ebenfalls in der Lage ist den Fluoreszenzfarbstoff zu verdrängen (jedoch weniger stark als Arginin). Die Reaktion wurde durchgeführt in Anwesenheit von 100 µM des Fluoreszenzfarbstoffs DBO-Amin und 200 µM *p*-Sulfonato-Calix[4]aren in Wasser bei pH 9,5.

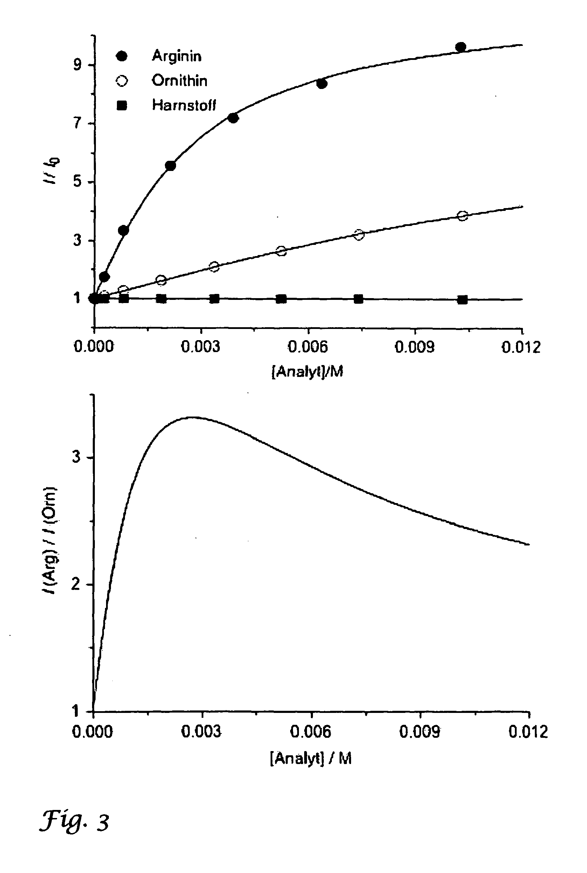

Figur 3 ist eine Darstellung des Zusammenhangs zwischen dem Verhältnis aus der Ausgangs-Fluoreszenzintensität und der Intensität nach vollständiger Umsetzung von *L*-Arginin zu *L-*Ornithin in der in Figur 1 und 2 gezeigten Reaktion. Abhängig von den Bindungskonstanten von Arginin (K = 3170±130 M⁻¹) und Ornithin (K = 190±70 M⁻¹) zum Makrozyklus findet bei gleicher Analytkonzentration eine unterschiedlich starke Verdrängung des Fluoreszenzfarbstoffs und damit eine unterschiedlich starke Erhöhung der Fluoreszenzintensität statt (siehe Abbildung oben). Aus dem Verhältnis dieser Fluoreszenzintensitäten (III₀) ergibt sich die maximal zu erwartende Änderung der Fluoreszenzintensität während der enzymatischen Reaktion (siehe angenäherte Funktion in Abbildung unten). Die Bindung des Harnstoffs kann aufgrund der extrem kleinen Bindungskonstante vernachlässigt werden (ungeladenes Molekül mit einem Kationenrezeptor). Die Bindungskonstanten wurden in Wasser bei pH 7 in Anwesenheit von 100 µM DBO-Amin und 200 µM p-Sulfonato-Calix[4]aren bestimmt.

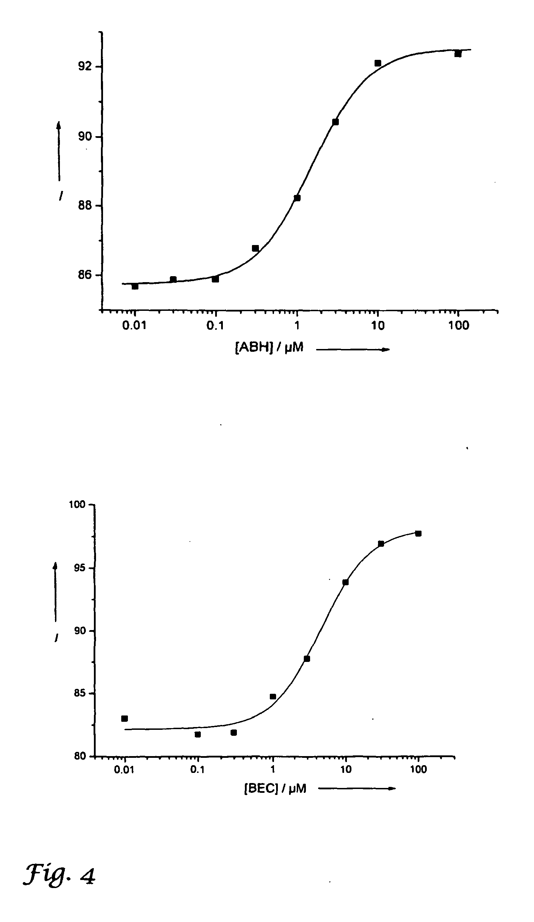

Figur 4 stellt eine Darstellung eines Inhibitor-Screenings dar. Es wurde eine Hill-Analyse zur Ermittlung der Effektivität von den Arginase-Inhibitoren a) 2(S)-Amino-6-boronohexansäure (ABH, Kᵢ = 1,0 ±0,1 µM) und b) *S*-(2-Boronoethyl)-*L-*cystein (BEC, Kᵢ = 4,9 ± 0,5 µM) durchgeführt. Dazu wurden 0,5 mM Arginin in Gegenwart unterschiedlicher Konzentrationen an den jeweiligen Inhibitoren mit 16 µg/ml Arginase umgesetzt. Die Lösung (Wasser, pH 9,5) enthielt ferner 100 µM DBO-Amin und 200 µM p-Sulfonato-Calix[4]aren. Zur Hilf-Analyse wurde die Fluoreszenzintensität nach 6 Minuten Inkubationszeit gegen den Logarithmus der Inhibitorkonzentration aufgetragen. Der Arginase-Assay ist damit zum Screenen einer Inhibitor-Bibliothek geeignet.

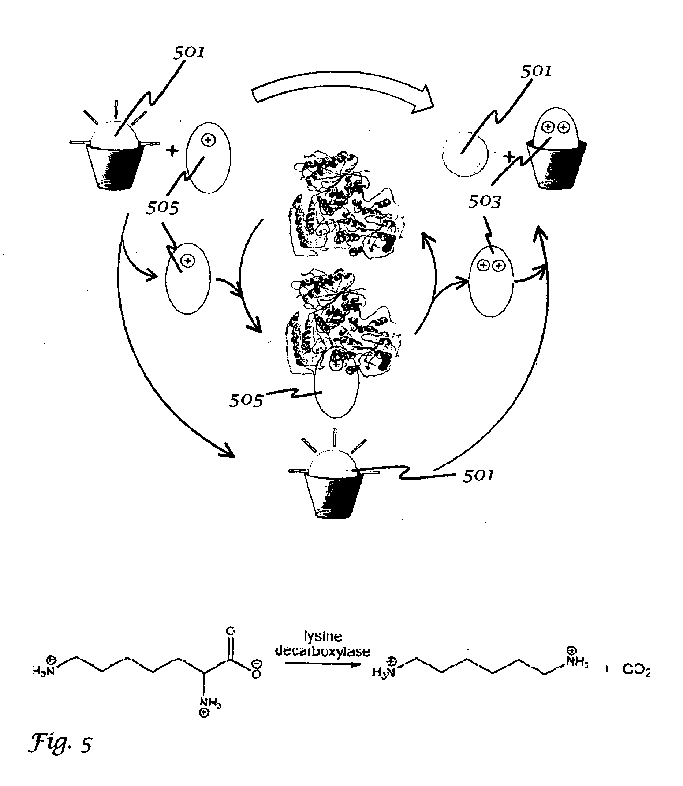

Figur 5 stellt ein Reaktionsschema der Umsetzung von *L*-Lysin **(505,** Analyt-Vorstufe) zu Cadaverin **(503,** Analyt, verdrängt den Fluoreszenzfarbstoff) und CO₂ (nicht gezeigt, entweicht größtenteils aus der Lösung) durch *L-*Lysin-Decarboxylase dar. Als Fluoreszenzfarbstoff wurde Dapoxyl **(501)** gewählt und Cucurbit[7]uril (ein Kationenrezeptor) wurde als Makrozyklus gewählt, wobei die Fluoreszenz von Dapoxyl **(501)** durch die Komplexierung mit Cucurbit[7]uril verstärkt wird. Infolge der Umsetzung der Analyt-Vorstufe **505** zum Analyten **503** nimmt die Fluoreszenz während der enzymatischen Reaktion ab, da immer mehr Farbstoff **501** durch den Analyten verdrängt wird und somit die Verstärkung der Dapoxyl-Fluoreszenz durch den Makrozyklus entfällt. Das Cucurbituril fungiert als Kationenrezeptor, der den zweifach positiv geladenen Analyten Cadaverin **503** besser bindet als die einfach positiv geladene (Gesamtladung) Analyt-Vorstufe *L-*Lysin **505.** Bei der schematisierten Darstellung des Enzyms Lysin-Decarboxylase handelt es sich ebenfalls um die in Fig. 1 verwendete Arginase, da für Lysin-Decarboxylase keine Kristallstruktur vorliegt. Die Darstellung erfolgte mit dem Programm Swiss-PdbViewer v3.7 (http://www.expasy.org/spdbv/.

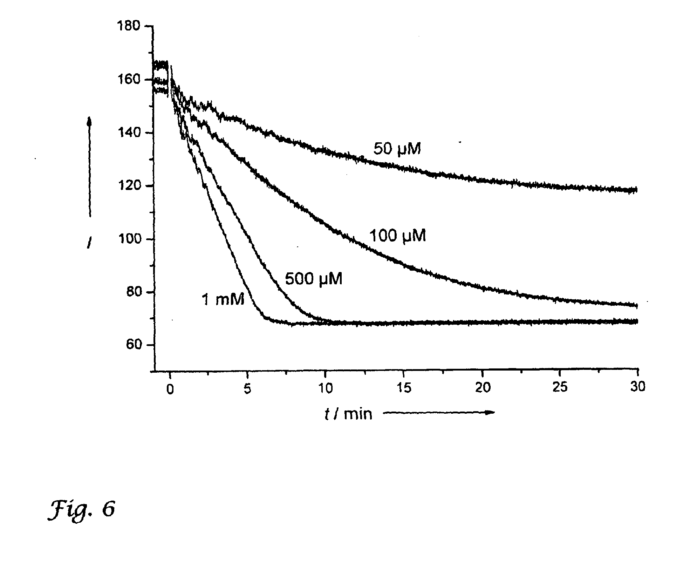

Figur 6 stellt die Fluoreszenzänderung von Lösungen unterschiedlicher Lysin-Konzentration nach Zugabe von 0.4 mg/ml Lysin-Decarboxylase (zum Zeitpunkt *t* = 0 Minuten) dar. Die Reaktion wurde in Gegenwart von 2.5 µM Dapoxyl und 200 µM Cucurbit[7]uril in einem 10 mM NH₄OAc-Puffer (pH 6.0) durchgeführt. Die enzymatische Reaktion kann somit in einfacher Weise ohne chemische Veränderung des *L-*Lysin, ohne radioaktive Markierung, und ohne Antikörper direkt in Lösung ohne weitere Inkubationsschritte mithilfe eines kommerziellen Fluoreszenzspektrometers (in diesem Fall: Varian Cary Eclipse, λ_{exc} = 336 nm, λ_{obs} = 390 nm) verfolgt werden. Gut zu erkennen ist in diesem Falle, dass die Bindungskonstanten von Lysin (K ca. 2000 M⁻¹) und Cadaverin (K > 1000000 M⁻¹) stark unterschiedlich sind. Somit hat im relevanten Konzentrationsbereich die Ausgangskonzentration von Lysin nur einen sehr geringen Einfluss auf die anfängliche Fluoreszenzintensität. Desweiteren ist zu erkennen, dass bereits 100 µM Cadaverin ausreichend sind, um den Fluoreszenzfarbstoff vollständig aus dem Makrozyklus zu verdrängen, d.h. im Falle von 500 µM und 1 mM Lysin können unter den gegebenen Bedingungen nur die ersten 20 bzw. 10 % der Reaktion verfolgt werden. Dies stellt insofern kein Problem dar, da im Allgemeinen zur Charakterisierung einer enzymatischen Reaktion sowieso nur die Anfangsgeschwindigkeit der Reaktion ermittelt wird. Das relativ starke Rauschen während der ersten 5 Minuten nach Zugabe des Enzyms ist darauf zurückzuführen, dass die Lysin-Decarboxylase als ungereinigter Zellextrakt verwendet wurde, und sich daher im Puffer unlösliche Bestandteile erst absetzen mussten.

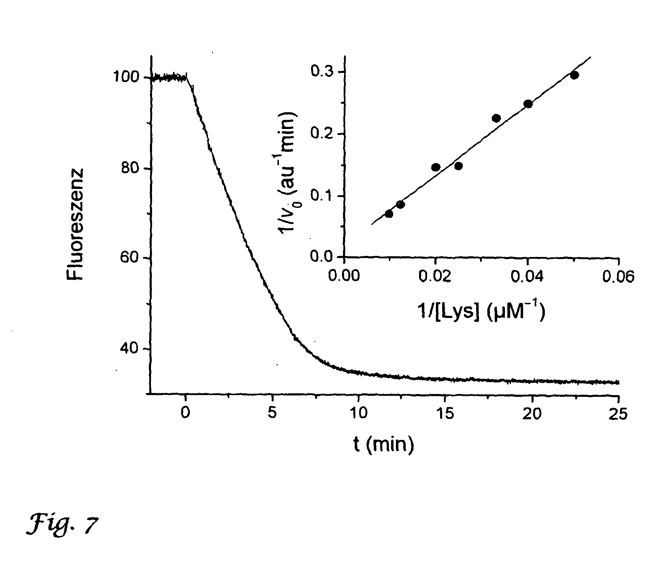

Figur 7 stellt die Bestimmung von Enzymkinetiken dar. Exemplarisch ist die Änderung der Fluoreszenzintensität einer Lösung von 100 µM Lysin, 2.5 µM Dapoxyl und 10 µM Cucurbit[7]uril in einem 10 mM NH₄OAc-Puffer (pH 6.0) nach Zugabe von 0.04 mg/ml Lysin-Decarboxylase (zum Zeitpunkt *t* = 0 Minuten) dargestellt. Durch Variation der Substratkonzentration (Lysin) lassen sich mit Hilfe der Methode von Lineweaver-Burk die kinetischen Parameter ermitteln. Anzumerken ist, dass im Vergleich zu Fig. 6 das Verhältnis von Makrozyklus/Farbstoff-Konzentration verändert wurde. Dadurch konnte eine geringere Enzymmenge eingesetzt/detektiert werden, so dass eine homogene Lösung vorlag. Desweiteren erlauben diese Bedingungen die Verwendung einer 10flach geringeren Substratkonzentration.

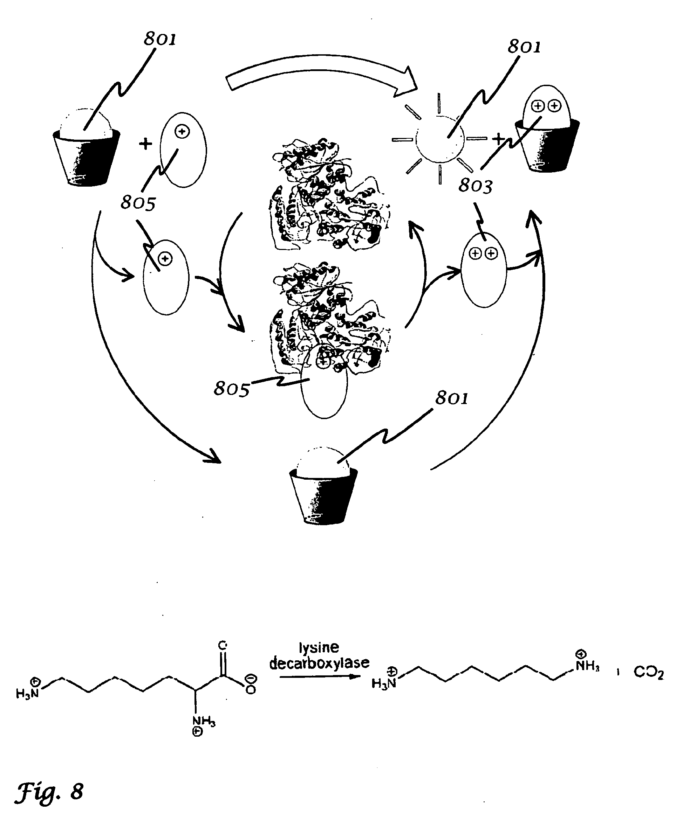

Figur 8 stellt ein Reaktionsschema der Umsetzung von *L*-Lysin **(805,** Analyt-Vorstufe) zu Cadaverin **(803,** Analyt, verdrängt den Fluoreszenzfarbstoff) und CO₂ (nicht gezeigt, entweicht größtenteils aus der Lösung) durch *L-*Lysin-Decarboxylase dar. Als Fluoreszenzfarbstoff wurde 1-Aminomethyl-2,3-diazabicyclo[2.2.2]oct-2-en **(801,** DBO-Amin) gewählt und p-Sulfonato-Calix[4]aren (ein Kationenrezeptor) wurde als Makrozyklus gewählt, wobei die Fluoreszenz von DBO-Amin **801** durch die Komplexierung mit dem Calixaren gelöscht wird. Infolge der Umsetzung der Analyt-Vorstufe **805** zum Analyten **803** nimmt die Fluoreszenz während der enzymatischen Reaktion zu, da immer mehr Farbstoff **801** durch den Analyten **803** verdrängt wird und somit die Löschung der DBO-Amin-Fluoreszenz durch den Makrozyklus entfällt. Das Calixaren fungiert als Kationenrezeptor, der den zweifach positiv geladenen Analyten Cadaverin **803** besser bindet als die einfach positiv geladene (Gesamtladung) Analyt-Vorstufe *L*-Lysin **805.** Bei der schematisierten Darstellung des Enzyms Lysin-Decarboxylase handelt es sich ebenfalls um die in Fig. 1 verwendete Arginase, da für Lysin-Decarboxylase keine Kristallstruktur vorliegt. Die Darstellung erfolgte mit dem Programm Swiss-PdbViewer v3.7 **(http://www.expasy.org/spdbv/).**

Dieses Beispiel veranschaulicht die breite Anwendbarkeit, da im Folgenden die gleiche Reaktion wie in Fig. 5-7 nachgewiesen wird, jedoch mit einem anderen System von Makrozyklus und Fluoreszenzfarbstoff.

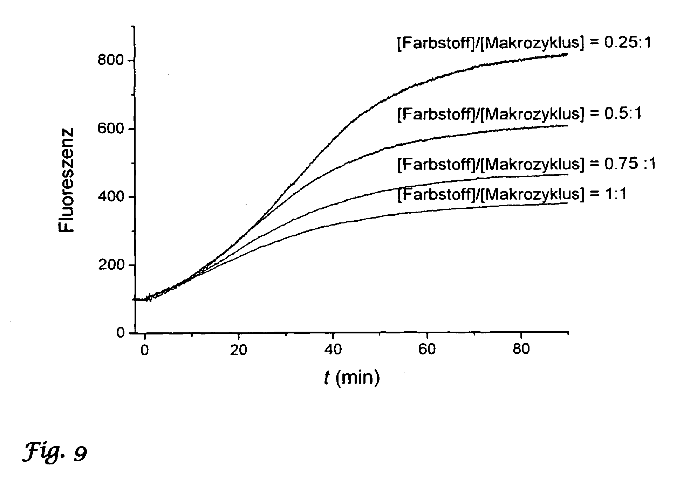

Figur 9 stellt die die Fluoreszenzänderung von Lösungen unterschiedlicher Farbstoff-/Makrozyklus-Konzentrationsverhältnisse nach Zugabe von 0.4 mg/ml Lysin-Decarboxylase (zum Zeitpunkt *t* = 0 Minuten) dar. Die Reaktion wurde in Gegenwart von 100-400 µM DBO-Amin und 400 µM Calixaren in einem 10 mM NH₄OAc-Puffer (pH 6.0) durchgeführt. Die Substratkonzentration betrug 500 µM. Gut zu erkennen ist in diesem Falle, dass unterschiedliche Versuchsbedingungen zu ungewöhnlichen Verlaufskurven führen können. So spiegelt in diesen Fällen die Fluoreszenzintensität nicht direkt (proportional) den enzymatischen Umsatz wider, sondern ist nur ein qualitatives Mass für die Enzymaktivität. Andererseits wird hiermit demonstriert, dass durch Variation der Randbedingungen z.B. der Anstieg der Fluoreszenzintensität weitergehend optimierbar ist.

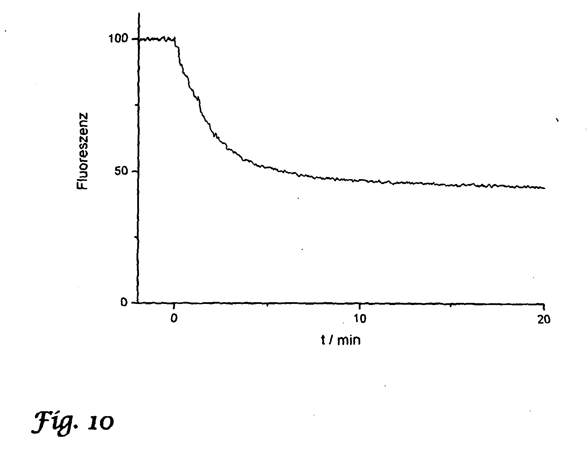

Figur 10 stellt die Möglichkeit dar ungereinigte Zellen anstelle eines gereinigten Enzyms zu untersuchen. Exemplarisch ist die Änderung der Fluoreszenzintensität einer Lösung von 150 µM Tyrosin, 2.5 µM Dapoxyl und 10 µM Cucurbit[7]uril in einem 10 mM NH₄OAc-Puffer (pH 6.0) nach Zugabe von 0.04 mg/ml getrockneter *Streptococcus faecalis-Zellen* (zum Zeitpunkt *t* = 0 Minuten) dargestellt. Die Zellen enthalten das Enzym Tyrosin-Decarboxylase, welches die Umsetzung von Tyrosin zu Tyramin katalysiert.

## Patentansprüche

1. Verfahren zum Bestimmen einer Konzentrationsänderung in Folge einer katalytischen Reaktion, insbesondere einer enzymatischen Reaktion, eines Analyten in einer Probe, umfassend die Schritte:
a) Bereitstellen eines Fluoreszenzfarbstoffs und eines Makrozyklus zu der zu untersuchenden Probe, wobei der Analyt den Fluoreszenzfarbstoff in dem zu untersuchenden Konzentrationsbereich des Analyten vom Makrozyklus verdrängt,
b) Messen einer Fluoreszenzeigenschaft des Fluoreszenzfarbstoffs an zumindest zwei Zeitpunkten und
c) ferner umfassend einen Reaktionspartner zum Umsetzen des Analyten, so dass der umgesetzte Analyt in dem zu untersuchenden Analyten-Konzentrationsbereich den Fluoreszenzfarbstoff nicht oder nicht so stark wie der Analyt vom Makrozyklus verdrängt, und/oder einen Vorläufer (Edukt) des Analyten, der zum Analyten umgesetzt wird, und der in dem zu untersuchenden Analyten-Konzentrationsbereich den Fluoreszenzfarbstoff nicht oder nicht so stark wie der Analyt vom Makrozyldus verdrängt.

2. Verfahren nach Anspruch 1, **dadurch gekennzeichnet, dass** der Reaktionspartner ein Katalysator ist.

3. Screening-Verfahren zum Suchen eines Reaktionspartners für einen Analyten, umfassend die Schritte: a) Bereitstellen einer Anzahl von Mischungen enthaltend den vorgewählten Analyten, einen zu testenden Reaktionspartner für den Analyten, einen Fluoreszenzfarbstoff und einen Makrozyklus, wobei der Analyt den Fluoreszenzfarbstoff in dem zu untersuchenden Konzentrationsbereich des Analyten vom Makrozyklus verdrängt, und b) Durchführen eines Verfahrens nach einem der vorherigen Ansprüche mit jeder der in a) bereitgestellten Mischungen.

4. Screening-Verfahren zum Suchen eines Inhibitors einer vorgewählten Reaktion eines Analyten, umfassend die Schritte: a) Bereitstellen einer Anzahl von Mischungen enthaltend den vorgewählten Analyten und den oder die zum Durchführen der vorgewählten Reaktion notwendigen Reaktionspartner, einen zu testenden Inhibitor der Reaktion, einen Fluoreszenzfarbstoff und einen Makrozyklus, wobei der Analyt den Fluoreszenzfarbstoff in dem zu untersuchenden Konzentrationsbereich des Analyten vom Makrozyklus verdrängt, und b) Durchführen eines Verfahrens nach einem der Ansprüche 1 oder 2 mit jeder der in a) bereitgestellten Mischungen.

5. High-Throughput-Screening-Verfahren, wobei das High-Throughput-Screening-Verfahren ein Screening-Verfahren nach einem der Ansprüche 3 oder 4 erfolgt, **dadurch gekennzeichnet, dass** in Schritt a) des jeweiligen Screening-Verfahrcns zumindest 48 Mischungen bereitgestellt werden.

6. Vorrichtung zum Bestimmen einer Konzentrationsänderung in Folge einer katalytischen Reaktion eines Analyten, umfassend eine Probe mit einem Fluoreszenzfarbstoff und einem Makrozyklus, wobei der Analyt den Fluoreszenzfarbstoff in dem zu untersuchenden Konzentrationsbereich des Analyten vom Makrozyklus verdrängt, und einen Enzym zum Umsetzen des Analyten, so dass der umgesetzte Analyt in dem zu untersuchenden Analyten-Konzentrationsbereich den Fluoreszenzfarbstoff nicht oder nicht so stark wie der Analyt vom Makrozyklus verdrängt, und/oder - einen Vorläufer (Edukt) des Analyten, der zum Analyten umgesetzt wird und der in dem zu untersuchenden Analyten-Konzentrationsbereich den Fluoreszenzfarbstoff nicht oder nicht so stark wie der Analyt vom Makrozyklus verdrängt, ferner umfassend eine Lichtquelle zum Anregen des Fluoreszenzfarbstoffs und einen Detektor zum Messen der vom Fluoreszenzfarbstoff ausgehenden Fluoreszenz und/oder der Lichtabsorption des Fluoreszenzfarbstoffs.

7. Vorrichtung nach Ansprüch 6, ferner umfassend einen Träger mit Aufnahmen für zumindest 48 zu untersuchende Proben.

8. Verwendung eines Fluoreszenzfarbstoffs und eines Makrozyklus zum Bestimmen einer Konzentrationsänderung in Folge einer katalytischen Reaktion eines Analyten, wobei der Analyt den Fluoreszenzfarbstoff in dem zu untersuchenden Konzentrationsbereich des Analyten vom Makrozyklus verdrängt.

9. Verwendung nach Anspruch 8 eines anionenbindenden Makrozyklus, vorzugsweise eines Calixarens mit kationischen Gruppen, mit einem Fluoreszenzfarbstoff zum Nachweisen und/oder Bestimmen einer Konzentrationsänderung eines Anions.

10. Verwendung nach Anspruch 8 eines kationenbindenden Makrozyklus, vorzugsweise eines Calixarens mit anionischen Gruppen und/oder eines Cucurbiturils, mit einem Fluoreszenzfarbstoff zum Nachweisen und/oder Bestimmen einer Konzentrationsänderung eines Kations.

11. Verwendung nach Anspruch 8 eines wasserstoffbrückenbildenden Makrozyklus, vorzugsweise eines Cyclodextrins, mit einem Fluoreszenzfarbstoffs zum Nachweisen und/oder Bestimmen einer Konzentrationsänderung eines wasserstoffbrückenbildenden Analyten.

12. Verwendung nach Anspruch 8 eines stereoselektiven Makrozyklus, vorzugsweise eines Cyclodextrins, mit einem Fluoreszenzfarbstoffs zum Nachweisen und/oder Bestimmen einer Konzentrationsänderung eines Stereoisomeren eines Analyten.

## Claims

1. A method fur determining a change in concentration of an analyte in a specimen, as a consequence of catalystic reaction, more specifically of an enzymatic reaction, including the following steps:
a) Providing the specimen to be studied with a fluorescent dye and a macrocycle, the analyte displacing the fluorescent dye in the analyte cnncentration range to be studied away from the macrocycle.
b) Measuring a fluorescence property nf the fluorescent dye at least at two different moments and
c) Furthermore including a reacting agent fur transforming the analyte, so that the transformed analyte in the analyte concentration range to he studied does nut displace the fluorescent dye as much as the analyte in the macrocycle, and/or a precursor (reactant) of the analyte which is transformed into the analyte and which does not displace the fluorescent dye away from the macrocycle in the analyte concentration range or not as much as the analyte.

2. The method according tn claim I, **characterized in that** the reacting agent is a catalyst.

3. A screening method for searching fur a reacting agent fur an analyte, including the following steps: a) providing a number nf mixtures containing the pre-selected analyte, a reacting agent to be tested for the analyte, a fluorescent dye and a macrocycle, the analyte displacing the fluorescent dye away from the macrocycle in the concentration range to be studied and b) implementing a method according to one of the aforementioned claims with each of the mixtures provided in a).

4. A screening method for searching for an inhibitor of a pre-selected reaction of an analyte, including the following steps: a) providing a number of mixtures containing the pre-seclected analyte and the reacting agent, necessary for implementing the pre-selected reaction, a reaction inhibitor to be tested, a fluorescent dye and a macrocycle, the analyte displacing the fluorescent dye from the macrocycle in the concentration range to be studied and b) implementing a method according to one of the claims 1 or 2 witch each of the mixtures provided in a).

5. High-throughput screening method, the high-throughput screening method is a screening method according to one of the claims 3 or 4, **characterized in that** at least 48 mixtures are provided in step a) of each screening method.

6. A device for determining a change of concentration as a consequence of a catalytic reaction of an analyte, including a specimen with a fluorescent dye and a macrocycle, the analyte displacing the fluorescent dye away from the macrocycle in the analyte concentration range to be tested, and an enzyme fur transforming the analyte, so that the transformed analyte in the analyte concentration range does not displace the fluorescent dye away from the macrocycle or not as much as the analyte and/or a precursor (reactant) of the analyte, which is transformed into the analyte and which does not displace the fluorescent in the analyte concentration range dye away from the macrocycle or not as much as the analyte, furthermore including a light source fur stimulating the fluorescent dye and a detector for measuring the fluorescence issuing from the fluorescent dye and/or the light absorption of the fluorescent dye.

7. The method according to claim 6, furthermore including a support with receptacle for all least 48 specimens to be tested.

8. A use of a fluorescent dye and a macrocycle for determining a charge in concentration as a consequence of a catalytic reaction of an analyte, the analyte displacing the fluorescent dye in the analyte concentration range to be tested away from the macrocycle.

9. The use according to claim 8 of an anion binding macrocycle, preferably of a calixarene with cationic groups, with a fluorescent dye for detecting or determining a change in concentration of an anion

10. The use according to claim 8 of a cation binding macrocycle, preferably a calixarene with anionic groups and/or a cucurbituril, with a fluorescent dye for detecting or determining a change in concentration of a cation.

11. The use according to claim 8 of a hydrogen bonding macrocycle, preferably a cyclodextrin, with a fluorescent dye for detecting and/or determining a change in concentration of a hydrogen bonding macrocycle.

12. The use according to claim 8 of a stereoselective macrocycle, preferably a cyclodextrin, with a fluorescent dye for detecting and/or determining a change in concentration of a stereoisomer of an analyte.

## Revendications

1. Méthode pour déterminer un changement de concentration à la suite d'une réaction catalytique, notamment d'une réaction enzymatique, d'un analyte d'un échantillon comportant les étapes suivantes :
a) Préparation d'un colorant fluorescent et d'un macrocycle pour l'échantillon à étudier, l'analyte évinçant le colorant fluorescent du macrocycle dans la plage de concentration de l'analyte.
b) Mesure d'une propriété fluorescente du colorant fluorescent à au moins deux moments et
c) Comportant de plus un partenaire de réaction pour transformer l'analyte, de sorte que dans la plage de concentration de l'analyte à étudier, l'analyte transformé n'évince pas le colorant fluorescent du macrocycle ou l'évince moins que l'analyte, et/ou un précurseur (réactif) de l'analyte qui est transformé en l'analyte et qui, dans la plage de concentration de l'analyte à étudier, n'évince pas le colorant fluorescent du macrocycle ou l'évince moins que l'analyte.

2. Méthode selon la revendication 1, **caractérisée en ce que** le partenaire de réaction est un catalyseur.

3. Méthode de screening pour chercher un partenaire de réaction pour un analyte comprenant les étapes suivantes : a) préparation d'un certain nombre de mélanges contenant l'analyte présélectionné, un partenaire de réaction à tester pour l'analyte, un colorant fluorescent et un macrocycle, l'analyte évinçant le colorant fluorescent, du macrocycle dans la plage de concentration de l'analyte à étudier et b) mise en oeuvre d'une méthode selon l'une quelconque des revendications précédentes avec chacun des mélanges préparés en a).

4. Méthode de criblage pour chercher un inhibiteur d'une réaction présélectionnée d'un analyte comprenant les étapes suivantes : a) préparation d'un certain nombre de mélanges contenant l'analyte présélectionné et le partenaire de réaction nécessaire à la mise en oeuvre de la réaction présélectionnée, un inhibiteur de la réaction à tester, un colorant, fluorescent, et un macrocycle, l'analyte évinçant, le colorant fluorescent du macrocycle dans la plage de concentration de l'analyte à étudier et b) mise en oeuvre d'une méthode selon l'une des revendications 1 ou 2 avec chacun des mélanges préparés en a).

5. Méthode de criblage à haut débit, la méthode de criblage à haut débit étant une méthode de criblage selon l'une des revendications 3 ou 4, **caractérisée en ce qu'**au moins 48 mélanges sont préparés à l'étape a) de la méthode de criblage respective.

6. Méthode pour déterminer un changement de concentration à la suite d'une réaction catalytique d'un analyte comprenant un échantillon avec un colorant fluorescent et un macrocycle, l'analyte évinçant le colorant fluorescent, du macrocycle dans la plage de concentration de l'analyte à étudier et un enzyme pour transformer l'analyte de sorte que dans la plage de concentration de l'analyte à étudier, l'analyte transformé n'évince pas le colorant fluorescent du macrocycle ou l'évince moins que l'analyte, et/ou un précurseur (réactif) de l'analyte qui est transformé en l'analyte et qui dans la plage de concentration de l'analyte à étudier, n'évince pas le colorant fluorescent du macrocycle ou l'évince moins que l'analyte, cnmportant de plus une source lumineuse pour stimuler le colorant fluorescent et un détecteur, pour mesurer la fluorescence émanant du colorant fluorescent et/ou l'absorption lumineuse du colorant fluorescent.

7. Méthode selon la revendication 6 comprenant de plus un support avec des logements pour recevoir au moins 48 mélanges à étudier.

8. Utilisation d'un colorant fluorescent et d'un macrocycle pour déterminer un changement de concentration à la suite d'une réaction catalytique d'un analyte, l'analyte évinçant le colorant fluorescent du macrocycle dans la plage de concentration de l'analyte à étudier.

9. Utilisation selon la revendication 8 d'un macrocycle liant les anions, de préférence un calixarène avec des groupes cationiques, avec un colorant fluorescent pour déceler et/ou déterminer un changement de concentration d'un anion.

10. Utilisation selon la revendication 8 d'un macrocycle liant les cations, de préférence un calixarène avec des groupes anioniques et/ou un cucurbituril, avec un colorant fluorescent pour déceler et/ou déterminer un changement de concentration d'un cation.

11. Utilisation selon la revendication d'un macrocycle formant des liaisons hydrogènes, de préférence une cyclodextrine, avec un colorant fluorescent pour déceler et/ou déterminer un changement de concentration d'un analyte formant, des liaisons hydrogènes.

12. Utilisation selon la revendication 8, d'un macrocycle stéréosélectif, de préférence une cyclodextrine, avec un colorant fluorescent pour déceler et/ou déterminer un changement, de concentration d'un stéréo-isomère d'un analyte.
